# EUROPEAN PATENT SPECIFICATION

(11) **EP 2 027 861 B1**
(45) Date of publication and mention of the grant of the patent: **31.08.2011**
(21) Application number: 08017191.1
(22) Date of filing: 13.07.2006
(51) Int. Cl.: A61K 31/473, A61P 25/14

(54) **Use of 3,11b-cis-dihydrotetrabenazine for the treatment of symptoms of Huntington's disease**
Verwendung von 3,11B-Cis-Dihydrotetrabenazine zur Behandlung von Symptomen von Huntington-Chorea
Utilisation de 3,11b-cis-dihydrotetrabenazine pour le traitement des symptomes de la maladie de Huntington

(30) Priority: 14.07.2005 GB 0514501
(43) Date of publication of application: 25.02.2009
(62) Divisional of application: 06764942.6
(73) Proprietor: Biovail Laboratories International (Barbados) SRL, Christ Church 17154 (BB)
(72) Inventor: Tridgett, Robert, Wallsend NE28 9NX (GB); Filloux, Thierry, 74800 La Roche sur Foron (FR)
(74) Representative: Hutchins, Michael Richard

(56) References cited:
- WO-A-2005/077946
- WO-A2-2006/053067
- KILBOURN M R ET AL: "Absolute Configuration of (+)-alpha-Dihydrotetrabenazine, an Active Metabolite of Tetrabenazine" CHIRALITY, WILEY-LISS, NEW YORK, US, vol. 9, no. 1, 1997, pages 59-62, XP002329921 ISSN: 0899-0042
- KILBOURN M ET AL: "Binding of alpha-dihydrotetrabenazine to the vesicular monoamine transporter is stereospecific" EUROPEAN JOURNAL OF PHARMACOLOGY, AMSTERDAM, NL, vol. 278, no. 3, 1995, pages 249-252, XP002329922 ISSN: 0014-2999

## Description

This invention relates to the use of dihydrotetrabenazine in treating Huntington's Disease.

### Background of the invention

Huntington's Disease, formerly known as Huntington's Chorea, is an inherited neuro-degenerative disease that is currently incurable. The disease is caused by a CAG trinucleotide repeat expansion (referred to as HD mutation) in the IT15 gene located on chromosome 4p 16.3 which produces an abnormal form of a protein named Huntingtin. The abnormal protein triggers a process that results in the death of neurons in the corpus striatum region of the brain, possibly by the clumping or aggregation of the abnormal protein inside many types of neurons.

The Huntington's disease (HD) gene comprises a segment of DNA which contains the repeating sequence of nucleotides CAG coding for the amino acid glutamine. It has been found that if there are thirty or fewer CAG repeats within the gene, a person carrying the gene will not contract HD. However, persons carrying a gene in which there are over forty CAG repeats do tend to contract the disease.

Huntington's disease is transmitted via an autosomal dominant inheritance pattern such that each child of an HD-affected parent has a 50% chance of inheriting the disorder. The symptoms of Huntington's disease typically appear between the ages of about 30 and 50 years and the disease usually progresses over a 10 - 25 year period. The characteristics and symptoms of the disease include personality changes, depression, mood swings, unsteady gait, involuntary chorea, twitching and jerking movements and tremors, dementia, slurred speech, impaired judgement, difficulty in swallowing and an intoxicated appearance.

Once an individual becomes symptomatic for Huntington's disease the course of the disease can last anywhere from ten to thirty years. Typically, the course of HD can be roughly divided into three stages, the early, middle and late stages,

In the early stage, stage patients can still perform most of their usual activities. They may still be working and may still be able to drive. Whilst they may exhibit slight uncontrollable movements, stumbling and clumsiness, lack of concentration, short-term memory lapses and depression, as well as mood swings, the involuntary movements are relatively mild, speech is still clear, and dementia, if present at all, is mild.

During the middle stage, patients become more disabled and typically need assistance with some of their routine daily activities. Falls, weight loss, and swallowing difficulties may be a problem during this stage and dementia becomes more obvious to the casual observer. In addition, the uncontrollable movements become more pronounced.

During the late stage, patients deteriorate to the point where they require almost total care and many require constant attention in hospitals or nursing homes. At this stage, they may no longer be able to walk or speak and, although they may show fewer involuntary movements, may become more rigid. Patients in this stage are often unable to swallow food. At this stage most patients lose insight and are apparently unaware of their surroundings. When the patient finally dies, the cause of death is usually related to the same natural causes that lead to death in other severely debilitated patients, such as malnutrition or pneumonia.

According to the US National Institute of Neurological Disorders and Stroke (NINDS), a part of the National Institute of Health (NIH), there is currently no way of stopping or reversing the course of Huntington's disease.

Attempts have been made to develop treatments for HD and one study by Karpuj et al in Nature Medicine, February 2002, vol.8, no.2, pp. 143 -149 has involved the administration of cystamine. Apparently, the cystamine inactivates the enzyme transglutaminase which helps to create the clumps of Huntingtin protein thought to be responsible for the disease. Nevertheless, at present, so far as the applicants are aware, there is currently no generally available medicine for treating or arresting the progression of Huntington's disease.

The discovery of the gene responsible for Huntington's disease (see the paper by the Huntington's Disease Collaborative Group, Cell, Vol. 72, March 26, 1993, p. 971) has enabled diagnostic tests for the presence of the mutant form of the gene to be developed. Diagnostic tests, which make use of the polymerase chain reaction (PCR) to detect the number of CAG repeats on the IT-15 gene, are now widely available and allow a prediction to be made whether or not a patient will develop the symptoms of Huntington's disease; see for example the review by M. Hayden et al., Am J. Hum. Genet. 55:606-617 (1994); the article by S. Hersch, "The Neurogenetics Genie: Testing for the Huntington's disease mutation." Neurol. 1994; 44:1369-1373; and the article by R. R. Brinkman et al. (1997) "The likelihood of being affected with Huntington disease by a particular age, for a specific CAG size", Am. J. Hum. Genet. 60:12A2-1210.

Tetrabenazine (Chemical name: 1,3,4,6,7,11b-hexahydro-9,10-dimethoxy-3-(2-methylpropyl)-2H-benzo(a)quinolizin-2-one) has been in use as a pharmaceutical drug since the late 1950s. Initially developed as an anti-psychotic, tetrabenazine is currently used in the symptomatic treatment of hyperkinetic movement disorders such as Huntington's disease, hemiballismus, senile chorea, tic, tardive dyskinesia and Tourette's syndrome, see for example Jankovic et al., Am. J Psychiatry. (1999) Aug; 156(8):1279-81 and Jankovic et al., Neurology (1997) Feb; 48(2):358-62.

The primary pharmacological action of tetrabenazine is to reduce the supply of monoamines (e.g. dopamine, serotonin, and norepinephrine) in the central nervous system by inhibiting the human vesicular monoamine transporter isoform 2 (hVMAT2), The drug also blocks postsynaptic dopamine receptors.

Tetrabenazine is an effective and safe drug for the treatment of a variety of hyperkinetic movement disorders and, in contrast to typical neuroleptics, has not been demonstrated to cause tardive dyskinesia. Nevertheless, tetrabenazine does exhibit a number of dose-related side effects including causing depression, Parkinsonism, drowsiness, nervousness or anxiety, insomnia and, in rare cases, neuroleptic malignant syndrome.

The central effects of tetrabenazine closely resemble those of reserpine, but it differs from reserpine in that it lacks activity at the VMAT1 transporter. The lack of activity at the VAT transporter means that tetrabenazine has less peripheral activity than reserpine and consequently does not produce VMAT1-related side effects such as hypotension.

The chemical structure of tetrabenazine is as shown in Figure 1 below.

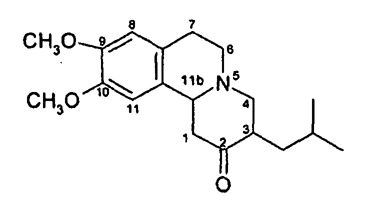

The compound has chiral centres at the 3 and 1 11b carbon atoms and hence can, theoretically, exist in a total of four isomeric forms, as shown in Figure 2.

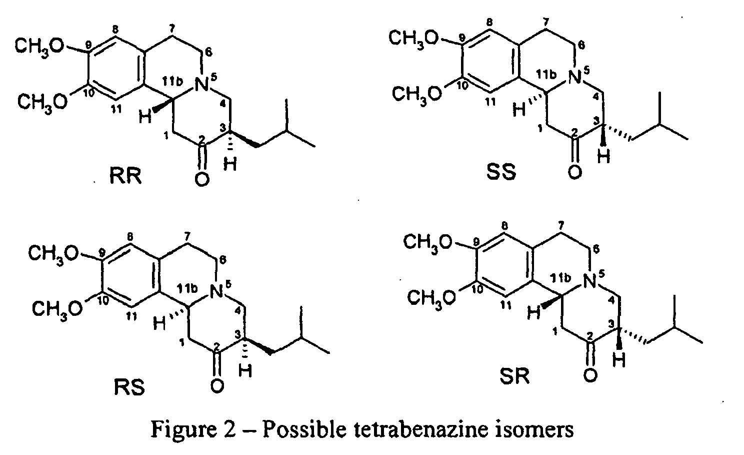

In Figure 2, the stereochemistry of each isomer is defined using the "R and S" nomenclature developed by Cahn, Ingold and Prelog, see Advanced Organic Chemistry by Jerry March, 4th Edition, John Wiley & Sons, New York, 1992, pages 109-114. In Figure 2 and elsewhere in this patent application, the designations "R" or "S" are given in the order of the position numbers of the carbon atoms. Thus, for example, *RS* is a shorthand notation for 3*R*,11*bS*. Similarly, when three chiral centres are present, as in the dihydrotetrabenazines described below, the designations "R" or "S" are listed in the order of the carbon atoms 2, 3 and 11 b. Thus, the 2*S*,3*R*,*11bR* isomer is referred to in short hand form as *SRR* and so on.

Commercially available tetrabenazine is a racemic mixture of the *RR* and *SS* isomers and it would appear that the *RR* and *SS* isomers (hereinafter referred to individually or collectively as *trans*-tetrabenazine because the hydrogen atoms at the 3 and 11b positions have a *trans* relative orientation) are the most thermodynamically stable isomers.

Tetrabenazine has somewhat poor and variable bioavailability. It is extensively metabolised by first-pass metabolism, and little or no unchanged tetrabenazine is typically detected in the urine. The major metabolite is dihydrotetrabenazine (Chemical name: 2-hydroxy-3-(2-methylpropyl)-1,3,4,6,7,1 1b-hexahydro-9,10-dirnethoxy-benzo(a)quinolizine) which is formed by reduction of the 2-keto group in tetrabenazine, and is believed to be primarily responsible for the activity of the drug (see Mehvar et al., Drug Metab.Disp, 15, 250-255 (1987) and J Pharm. Sci., 76, No.6, 461-465 (1987)), and Robert et al., Eur. J. Clin. Pharmacol., 29. 703-708 (1986).

Four dihydrotetrabenazine isomers have previously been identified and characterised, all of them being derived from the more stable RR and SS isomers of the parent tetrabenazane and having a *trans* relative orientation between the hydrogen atoms at the 3 and 11b positions) (see Kilboum et al., Chirality, 9:59-62 (1997) and Brossi et al., Hetv, Chim. Acta., vol. XLI, No. 193, ppl 793-1806 (1958). The four isomers are (+)-α-dihydroten:abenazine, (-)-α-dihydrotetrabenazine, (+)-β-dihydrotetrabenazine and (-)-β-dihydrotetrabenazine. The structures of the four known dihydrotetrabenazine isomers are considered to be as shown in Figure 3.

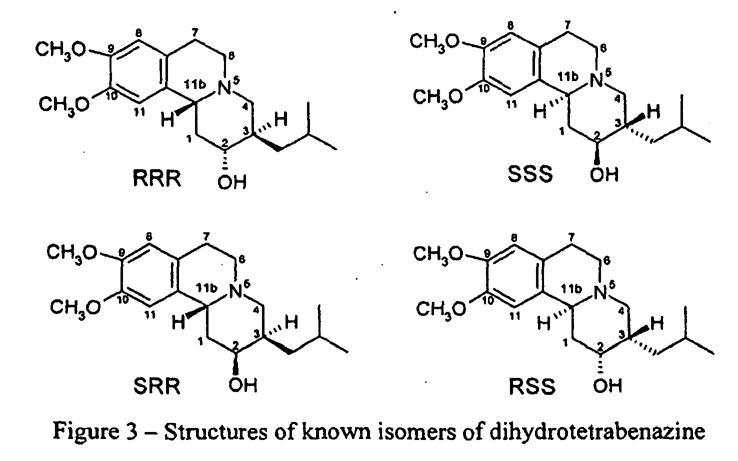

Kilbourn et al.,(see Eur. J. Pharmacol., 278:249-252 (1995) and Med. Chem. Res., 5:113-126 (1994)) investigated the specific binding of individual radio-labelled dihydrotetrabenazine isomers in the conscious rat brain. They found that the (+)-α-[¹¹C]dihydrotetrabenazine (2*R*,3*R,*11b*R*) isomer accumulated in regions of the brain associated with higher concentrations of the neuronal membrane dopamine transporter (DAT) and the vesicular monoamine transporter (VMAT2). However, the essentially inactive (-)-α-[¹¹C]dihydrotetrabenazine isomer was almost uniformly distributed in the brain, suggesting that specific binding to DAT and VMAT2 was not occurring. The *in vivo* studies correlated with *in vitro* studies which demonstrated that the (+)-α-[¹¹C]dihydrotetrabenazine isomer exhibits a Kᵢ for [³H]methoxytetrabenazine >2000-fold higher than the Kᵢ for the (-)-α-[¹¹C]dihydrotetrabenazine isomer.

International patent application number PCT/GB2005/000464 (Publication number WO 2005/077946) discloses the preparation and use of pharmaceutical dihydrotetrabenazine isomers derived from the unstable *RS* and *SR* isomers (hereinafter referred to individually or collectively as *cis*-tetrabenazine because the hydrogen atoms at the 3 and 11b positions have a *cis* relative orientation) of tetrabenazine.

### Summary of the Invention

It has now been found that the *cis*-dihydrotetrabenazine described in our earlier application no. PCT/GB2005/000464 has the ability to arrest or slow down the development of at least some of the symptoms of Huntington's disease. More particularly, it has been found that the deterioration of gait and the increase in involuntary movements (e.g. tremors and twitches) associated with Huntington's disease can be arrested or considerably slowed down by the administration of the *cis*-dihydrotetrabenazines of the invention.

Accordingly, in a first aspect, the invention provides 3,11b-*cis-*dihydrotetrabenazine for use in halting or slowing the progress of one or more symptoms of Huntington's disease selected from involuntary movements and degeneration in gait.

In another aspect, the invention provides the use of 3, 11 -*cis*-dihydrotetrabenazine for the manufacture of a medicament for halting or slowing the progress of one or more symptoms of Huntington's disease selected from involuntary movements and gait degeneration.

In one aspect, the symptoms are involuntary movements selected from involuntary chorea, tremors and twitches.

Diagnostic tests are currently available for determining whether an individual is carrying the mutant Huntington's disease gene. Since a person carrying the mutant gene will almost invariably develop Huntington's disease, it would be advantageous if the onset or development of the disease could be prevented, arrested or slowed down during the period in a person's life at which he or she is most likely to develop the disease.

Accordingly, in a further aspect of the invention, there is provided 3, 1 1b-*cis-*dihydrotetrabenazine for use in the prophylactic treatment of a patient identified as carrying the mutant gene responsible for Huntington's disease.

In another aspect of the invention, there is provided 3, 1 1b-*cis*-dihydrotetrabenazine for use in the prophylactic treatment of a patient identified as carrying the mutant gene responsible for Huntington's disease, wherein the prophylactic treatment is for preventing or slowing down sub-clinical progression of the disease in a patient. By sub-clinical progression is meant the development of the disease prior to the point at which the symptoms of the disease become apparent by clinical as opposed to biochemical investigation or investigation using scanning techniques such as computerised tomography or magnetic resonance imaging (MRI).

In another aspect, the invention provides 3,11b-*cis*-dihydrotetrabenazine for use in the prophylactic treatment of a patient within the age range 15-50 years, e.g. 20 - 50 years or 25 - 50 years or 30 - 50 years, who is carrying the mutant form of the HD gene but who has not yet developed symptoms of the disease, the prophylactic treatment being for the purpose of preventing or slowing the onset of symptoms associated with Huntington's disease.

References to the mutant form of the Huntington's Disease gene or like expressions in this application refer to forms of the gene in which the number of CAG repeats on the IT-15 gene is at least thirty five, more typically at least forty, for example at least 45, or at least 50. In some cases, there may be a very high number of CAG repeats (e.g. 70 or above) and persons carrying a mutant form of the gene with such a large number of CAG repeats is likely to develop the juvenile-onset form of the disease.

In a further aspect therefore, the *cis*-dihydrotetrabenazine may be administered prophylactically to persons of less than 30 years in age, for example in the range 10-29, more typically 15-29 or 20-29 years in age who have been tested and have been found to have mutant forms of the IT-15 gene in which the number of CAG repeats exceeds 60, and more particularly is at least 65, and preferably is 70 or more.

The *cis*-dihydrotetrabenazine used in the present invention is 3, 11b, *cis-*dihydrotetrabenazine.

The 3,11b-*cis*-dihydrotetrabenazine used in the invention may be in substantially pure form, for example at an isomeric purity of greater than 90%, typically greater than 95% and more preferably greater than 98%.

The term "isomeric purity" in the present context refers to the amount of 3,11b-*cis-*dihydrotetrabenazine present relative to the total amount or concentration of dihydrotetrabenazine of all isomeric forms. For example, if 90% of the total dihydrotetrabenazine present in the composition is 3,11b-*cis*-dihydrotetrabenazine, then the isomeric purity is 90%.

The 1 1b-*cis*-dihydrotetrabenazine used in the invention may be in the form of a composition which is substantially free of 3,11b-*trans*-dihydrotetrabenazine, preferably containing less than 5% of 3,1 1b-trans-dihydrotetrabenazine, more preferably less than 3% of 3,11b-trans-dihydrotetrabenazine, and most preferably less than 1% of 3,11b-*trans*-dibydroletrobenazine.

The term "3,11b-*cis*-" as used herein means that the hydrogen atoms at the 3- and 11 b-positions of the dihydrotetrabenazine structure are in the cis relative orientation. The isomers of the invention are therefore compounds of the formula (I) and antipodes (mirror images) thereof. 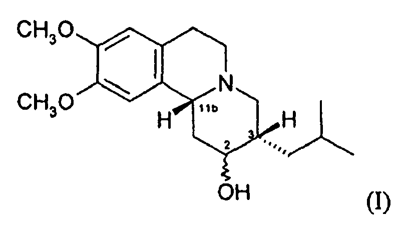 There are four possible isomers of dihydrotetrabenazine having the 3,11 *b-cis* configuration and these are the *2S,3S,*11 b*R* isomer, the 2*R*,3*R*, 11bS isomer, the *2R,*3*S,*11b*R* isomer and the 2*S*,3*R*,11b*S* isomer. The four isomers have been isolated and characterised and, in another aspect, the invention provides individual isomers of 3,1 1b-cis-dihydrotetrabenazine. In particular, the invention provides:
(a) the 2*S*,3*S*,11b*R* isomer of 3,11b-*cis*-dihydrotetrabenazine having the formula (Ia): 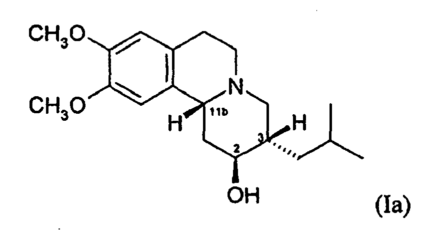
(b) the 2*R*,3*R*,11b*S* isomer of 3,11b-*cis*-dihydrotetrabenazine having the formula Ib): 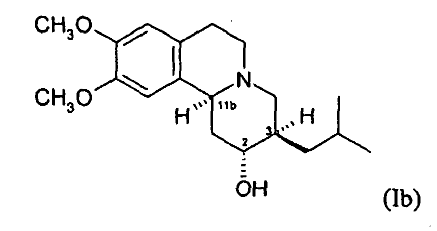
(c) the 2*R*,3*S*,11b*R* isomer of 3,11b-*cis*-dihydrotetrabenazine having the formula (Ic): 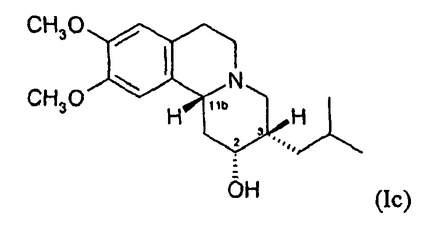 and
(d) the 2*S*,3*R*,11b*S* isomer of 3,11b-*cis*-dihydrotetrabenazine having the formula (Id):

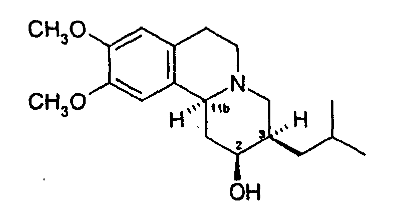

The individual isomers of the invention can be characterised by their spectroscopie, optical and chromatographic properties, and also by their absolute stereochemical configurations as determined by X-ray crystallography.

Preferred isomers are the dextrorotatory (+) isomers.

A particularly preferred isomer is isomer (la), i.e. the 2*S*,3*S*,11b*R* isomer of 3,11b-*cis*-dihydrotetrabenazine.

Without implying any particular absolute configuration or stereochemistry, the four novel isomers may be characterised as follows:

### Isomer A

Optical activity as measured by ORD (methanol, 21°C): laevorotatory (-) IR Spectrum (KBr solid), H-NMR spectrum (CDCl₃) and ¹³C-NMR spectrum (CDCl₃) substantially as described in Table 1.

### Isomer B

Optical activity as measured by ORD (methanol, 21°C): dextrorotatory (+) IR Spectrum (KBr solid), ¹H-NMR spectrum (CDCl₃) and ¹³C-NMR spectrum (CDCl₃) substantially as described in Table 1, and X-ray crystallographic properties as described in Example 4.

### Isomer C

Optical activity as measured by ORD (methanol, 21°C): dextrorotatory (+) IR Spectrum (KBr solid), ¹H-NMR spectrum (CDCl₃) and ³C-NMR spectrum (CDCl₃) substantially as described in Table 2.

### Isomer D

Optical activity as measured by ORD (methanol, 21°C): laevorotatory (-) IR Spectrum (KBr solid), ¹H-NMR spectrum (CDCl₃) and ¹³C-NMR Spectrum (CDCl₃) substantially as described in Table 2.

ORD values for each isomer are given in the examples below but it is noted that such values are given by way of example and may vary according to the degree of purity of the isomer and the influence of other variables such as temperature fluctuations and the effects of residual solvent molecules.

The enantiomers A, B, C and D may each be presented in a substantially enantiomerically pure form or as mixtures with other enantiomers of the invention.

The terms "enantiomeric purity" and "enantiomerically pure" in the present context refer to the amount of a given enantiomer of 3,11 b-cis-dihydrotetrabenazine present relative to the total amount or concentration of dihydrotetrabenazine of all enantiomeric and isomeric forms. For example, if 90% of the total dihydrotetrabenazine present in the composition is in the form of a single enantiomer, then the enantiomeric purity is 90%.

By way of example, in each aspect and embodiment of the invention, each individual enantiomer selected from Isomers A, B, C and D may be present in an enantiomeric purity of at least 55% (e.g. at least 60%, 65%, 70%, 75%, 80%, 85%, 90%, 95%, 97%, 98%, 99%, 99.5% or 100%).

The isomers of the invention may also be presented in the form of mixtures of one or more of Isomers A, B, C and D. Such mixtures may be racemic mixtures or non-racemic mixtures. Examples of racemic mixtures include the racemic mixture of Isomer A and Isomer B and the racemic mixture of Isomer C and Isomer D.

### Pharmaceutically Acceptable Salts

Unless the context requires otherwise, a reference in this application to dihydrotetrabenazine and its isomers, includes within its scope not only the free base of the dihydrotetrabenazine but also its salts, and in particular acid addition salts.

Particular acids from which the acid addition salts are formed include acids having a pKa value of less than 3.5 and more usually less than 3. For example, the acid addition salts can be formed from an acid having a pKa in the range from +3.5 to -3.5.

Preferred acid addition salts include those formed with sulphonic acids such as methanesulphonic acid, ethanesulphonic acid, benzene sulphonic acid, toluene sulphonic acid, camphor sulphonic acid and naphthalene sulphonic acid.

One particular acid from which acid addition salts may be formed is methanesulphonic acid.

Acid addition salts can be prepared by the methods described herein or conventional chemical methods such as the methods described in Pharmaceutical Salts: Properties, Selectiom and Use, P. Heinrich Stahl (Editor), Camille G. Wermuth (Editor), ISBN: 3-90639-026-8, Hardcover, 388 pages, August 2002. Generally, such salts can be prepared by reacting the free base form of the compound with the appropriate base or acid in water or in an organic solvent, or in a mixture of the two; generally, nonaqueous media such as ether, ethyl acetate, ethanol, isopropanol, or acetonitrile are used.

The salts are typically pharmaceutically acceptable salts. However, salts that are not pharmaceutically acceptable may also be prepared as intermediate forms which may then be converted into pharmaceutically acceptable salts. Such non-pharmaceutically acceptable salt forms also form part of the invention.

### Methods for the Preparation of Dihydrotetrabenazine Isomers

The dihydrotetrabenazine of the invention can be prepared by a process comprising the reaction of a compound of the formula (II): 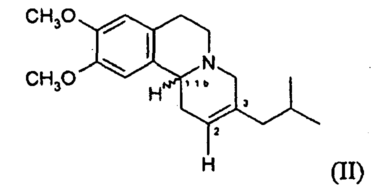 with a reagent or reagents suitable for hydrating the 2,3-double bond in the compound of formula (II) and thereafter where required separating and isolating a desired dihydrotetrabenazine isomer form.

The hydration of the 2,3-double bond can be carried out by hydroboration using a borane reagent such as diborane or a borane-ether (e.g. borane-tetrahydrofuran (THF)) to give an intermediate alkyl borane adduct followed by oxidation of the alkyl borane adduct and hydrolysis in the presence of a base. The hydroboration is typically carried out in a dry polar non-protic solvent such as an ether (e.g. THF), usually at a non-elevated temperature, for example room temperature. The borane-alkene adduct is typically oxidised with an oxidising agent such as hydrogen peroxide in the presence of a base providing a source of hydroxide ions, such as ammonium hydroxide or an alkali metal hydroxide, e.g. potassium hydroxide or sodium hydroxide. The hydroboration-oxidation-hydrolysis sequence of reactions of Process A typically provides dihydrotetrabenazine isomers in which the hydrogen atoms at the 2- and 3-positions have a *trans* relative orientation.

Compounds of the formula (II) can be prepared by reduction of tetrabenazine to give a dihydrotetrabenazine followed by dehydration of the dihydrotetrabenazine.

Reduction of the tetrabenazine can be accomplished using an aluminium hydride reagent such as lithium aluminium hydride, or a borohydride reagent such as sodium borohydride, potassium borohydride or a borohydride derivative, for example an alkyl borohydride such as lithium tri-sec-butyl borohydride. Alternatively, the reduction step can be effected using catalytic hydrogenation, for example over a Raney nickel or platinum oxide catalyst. Suitable conditions for performing the reduction step are described in more detail below or can be found in US 2,843,591 (Hoffmann- La Roche) and Brossi et al., Helv. China. Acta., vol, XLI, No. 193, pp1793-1806 (1958).

Because the tetrabenazine used as the starting material for the reduction reaction is typically a mixture of the *RR* and *SS* isomers (i.e. *trans*-tetrabenazine), the dihydrotetrabenazine formed by the reduction step will have the same *trans* configuration about the 3- and 11b positions and will take the form of one or more of the known dihydrotetrabenazine isomers shown in Figure 3 above. Thus Process A may involve taking the known isomers of dihydrotetrabenazine, dehydrating them to form the alkene (II) and then "dehydrating" the alkene (II) using conditions that give the required novel *cis* dihydrotetrabenazine isomers of the invention.

Dehydration of the dihydrotetrabenazine to the alkene (II) can be carried out using a variety of standard conditions for dehydrating alcohols to form alkenes, see for example J. March (*idem*) pages 389-390 and references therein. Examples of such conditions include the use of phosphorus-based dehydrating agents such as phosphorus halides or phosphorus oxyhalides, e.g. POCl₃ and PCl₅. As an alternative to direct dehydration, the hydroxyl group of the dihydrotetrabenazine can be converted to a leaving group L such as halogen (e.g. chlorine or bromine) and then subjected to conditions (e.g. the presence of a base) for eliminating H-L. Conversion of the hydroxyl group to a halide can be achieved using methods well known to the skilled chemist, for example by reaction with carbon tetrachloride or carbon tetrabromide in the presence of a trialkyl or triaryl phosphine such as triphenyl phosphine or tributyl phosphine.

The tetrabenazine used as the starting material for the reduction to give the dihydrotetrabenazine can be obtained commercially or can be synthesised by the method described in US 2,830,993 (Hoffmarin-La Roche).

Another process (Process B) for preparing a dihydrotetrabenazine of the invention comprises subjecting a compound of the formula (III); 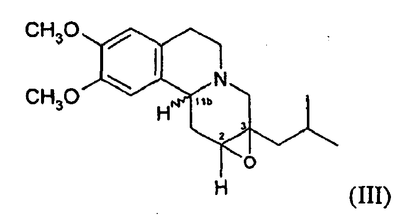 to conditions for ring-opening the 2,3-epoxide group in the compound of the formula (III), and thereafter where required separating and isolating a desired dihydrotetrabenazine isomer form.

The ring-opening can be effected in accordance with known methods for epoxide ring openings. However, a currently preferred method of ring-opening the epoxide is reductive ring opening which can be achieved using a reducing agent such as borane-THF. Reaction with borane-THF can be carried out in a polar non-protic solvent such as ether (e.g. tetrahydrofuran) usually at ambient temperature, the borane complex thus formed being subsequently hydrolysed by heating in the presence of water and a base at the reflux temperature of the solvent. Process B typically gives rise to dihydrotetrabenazine isomers in which the hydrogen atoms at the 2- and 3-positions have a cis relative orientation.

The epoxide compounds of the formula (III) can be prepared by epoxidation of an alkene of the formula (II) above. The epoxidation reaction can be carried out using conditions and reagents well known to the skilled chemist, see for example J. March *(idem),* pages 826-829 and references therein. Typically, a per-acid such as meta-chloroperbenzoic acid (MCPBA), or a mixture of a per-acid and a further oxidising agent such as perchloric acid, may be used to bring about epoxidation.

When the starting materials for processes A and B above are mixtures of enantiomers, then the products of the processes will typically be pairs of enantiomers, for example racemic mixtures, possibly together with diastereoisomeric impurities. Unwanted diastereoisomers can be removed by techniques such as chromatography (e.g. HPLC) and the individual enantiomers can be separated by a variety of methods known to the skilled chemist. For example, they can be separated by means of:
(i) chiral chromatography (chromatography on a chiral support); or
(ii) forming a salt with an optically pure chiral acid, separating the salts of the two diastereoisomers by fractional crystallisation and then releasing the dihydrotetrabenazine from the salt; or
(iii) forming a derivative (such as an ester) with an optically pure chiral derivatising agent (e.g. esterifying agent), separating the resulting epimers (e.g. by chromatography) and then converting the derivative to the dihydrotetrabenazine.

One method of separating pairs of enantiomers obtained from each of Processes A and B, and which has been found to be particularly effective, is to esterify the hydroxyl group of the dihydrotetrabenazine with an optically active form of Mosher's acid, such as the R (+) isomer shown below, or an active form thereof:

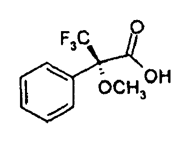

The resulting esters of the two enantiomers of the dihydrobenazine can then be separated by chromatography (e,g. HPLC)) and the separated esters hydrolysed to give the individual dibydrobenazine isomers using a base such as an alkali metal hydroxide (e.g. NaOH) in a polar solvent such as methanol.

As an alternative to using mixtures of enantiomers as the starting materials in processes A and B and then carrying out separation of enantiomers subsequently, processes A and B can each be carried out on single enantiomer starting materials leading to products in which a single enantiomer predominates. Single enantiomers of the alkene (II) can be prepared by subjecting RR/SS tetrabenazine to a stereoselective reduction using lithium tri-sec-butyl borohydride to give a mixture of SRR and RSS enantiomers of dihydrotetrabenazine, separating the enantiomers (e.g. by fractional crystallisation) and then dehydrating a separated single enantiomer of dihydrotetrabenazine to give predominantly or exclusively a single enantiomer of the compound of formula (II).

Processes A and B are illustrated in more detail below in Schemes 1 and 2 respectively.

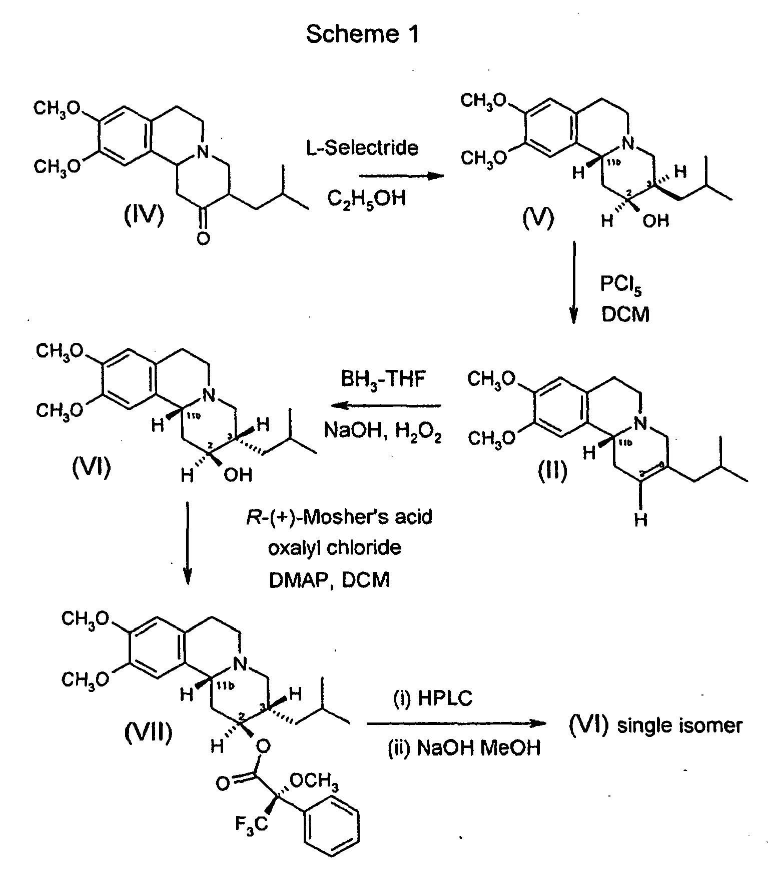

Scheme I illustrates the preparation of individual dihydrotetrabenazine isomers having the 2*S*,3*S* 11b*R*. and 2*R*,3*R*, 11b*S* configurations in which the hydrogen atoms attached to the 2- and 3-positions are arranged in a *trans* relative orientation. This ' reaction scheme includes Process A defined above.

The starting point for the sequence of reactions in Scheme 1 is commercially available tetrabenazine (IV) which is a racemic mixture of the RR and SS optical isomers of tetrabenazine. In each of the RR and SS isomers, the hydrogen atoms at the 3- and 1 lb-positions are arranged in a *trans* relative orientation. As an alternative to using the commercially available compound, tetrabenazine can be synthesised according to the procedure described in US patent number 2,830,993 (see in particular example 11).

The racemic mixture of RR and SS tetrabenazine is reduced using the borohydride reducing agent lithium tri-*sec*-butyl borohydride ("L-Selectride") to give a mixture of the known 2S,3R, 11bR and 2*R*,3*S*,11b*S* isomers (V) of dihydrotetrabenazine, of which only the 2*S*,3*R*, 11b*R* isomer is shown for simplicity. By using the more sterically demanding L-Selectride as the borohydride reducing agent rather than sodium borohydride, formation of the RRR and SSS isomers of dihydrotetrabenazine is minimised or suppressed.

The dihydrotetrabenazine isomers (V) are reacted with a dehydrating agent such as phosphorus pentachloride in a non-protic solvent such as a chlorinated hydrocarbon (for example chloroform or dichloromethane, preferably dichloromethane) to form the unsaturated compound (II) as a pair ofenantiomers, only the *R*-enantiomer of which is shown in the Scheme. The dehydration reaction is typically carried out at a temperature lower than room temperature, for example at around 0-5°C.

The unsaturated compound (II) is then subjected to a stereoselective re-hydration to generate the dihydrotetrabenazine (VI) and its mirror image or antipode (not shown) in which the hydrogen atoms at the 3- and 11 b-position are arranged in a *cis* relative orientation and the hydrogen atoms at the 2- and 3-positions are arranged in a *trans* relative orientation. The stereoselective rehydration is accomplished by a hydroboration procedure using borane-THF in tetrahydrofuran (THF) to form an intermediate borane complex (not shown) which is then oxidised with hydrogen peroxide in the presence of a base such as sodium hydroxide.

An initial purification step may then be carried out (e.g. by HPLC) to give the product (V) of the rehydration reaction sequence as a mixture of the 2*S*,3*S*,11b*R* and 2*R*,3*R*,11b*S* isomers of which only the 2*S*,3*S*, 11b*R* isomer is shown in the Scheme. In order to separate the isomers, the mixture is treated with *R* (+) Mosher's acid, in the presence of oxalyl chloride and dimethylaminopyridine (DMAP) in dichloromethane to give a pair of diastereoisomeric esters (VII) (of which only one diastereoisomer is shown) which can then be separated using HPLC. The individual esters can then be hydrolysed using an alkali metal hydroxide such as sodium hydroxide to give a single isomer (VI).

In a variation of the sequence of steps shown in Scheme 1, following the reduction of RR/SS tetrabenazine, the resulting mixture of enantiomers of the dihydrotetrabenazine (V) can be separated to give the individual enantiomers. Separation can be carried out by forming a salt with a chiral acid such as (+) or (-) camphorsulphonic acid, separating the resulting diastereoisomers by fractional crystallisation to give a salt of a single enantiomer and then releasing the free base from the salt.

The separated dihydrotetrabenazine enantiomer can be dehydrated to give a single enantiomer of the alkene (II). Subsequent rehydration of the alkene (II) will then give predominantly or exclusively a single enantiomer of the cis-dihydrotetrabenazine (VI). An advantage of this variation is that it does not involve the formation of Mosher's acid esters and therefore avoids the chromatographic separation typically used to separate Mosher's acid esters.

Scheme 2 illustrates the preparation of individual dihydrotetrabenazine isomers having the 2*R*,3*S*, 11bR and 2*S*,3*R*, 11b*S* configurations in which the hydrogen atoms attached to the 2- and 3-positions are arranged in a cis relative orientation. This reaction scheme includes Process B defined above.

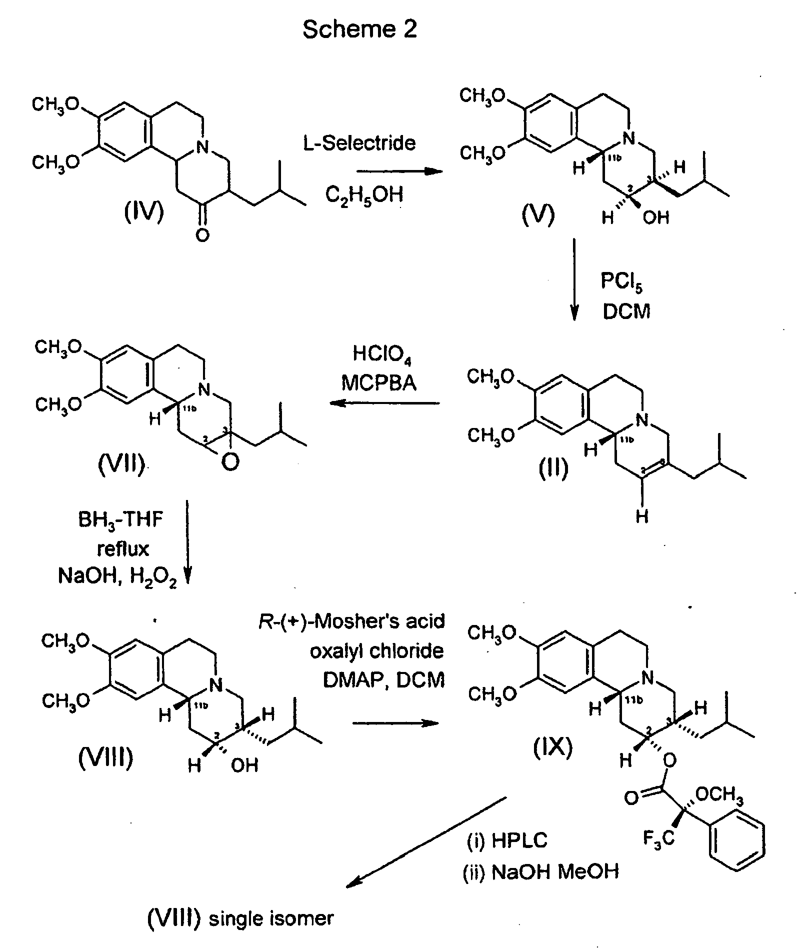

In Scheme 2, the unsaturated compound (II) is produced by reducing tetrabenazine to give the 2S,3R,11bR and 2R,3S,11bS isomers (V) of dihydrotetrabenazine and dehydrating with PCl₅ in the manner described above in Scheme 1. However, instead of subjecting the compound (11) to hydroboration, the 2,3-double bond is converted to an epoxide by reaction with *meta*-chloroperbenzoic acid (MCPBA) and perchloric acid. The epoxidation reaction is conveniently carried out in an alcohol solvent such as methanol, typically at around room temperature.

The epoxide (VII) is then subjected to a reductive ring opening using borane-THF as an electrophilic reducing agent to give an intermediate borane complex (not shown) which is then oxidised and cleaved with hydrogen peroxide in the presence of an alkali such as sodium hydroxide to give a dihydrotetrabenazine (VIII) as a mixture of the 2*R*,3*S*, 11b*R* and 2*S*,3*R*, 11b*S* isomers, of which only the 2*R*,3*S*, 1 1bR is shown for simplicity. Treatment of the mixture of isomers (VIII) with *R* (+) Mosher's acid in the presence of oxalyl chloride and dimethylaminopyridine (DMAP) in dichloromethane gives a pair of epimeric esters (IX) (of which only one epimer is shown) which can then by separated by chromatography and hydrolysed with sodium hydroxide in methanol in the manner described above in reflation to Scheme 1.

### Biological Properties and Therapeutic Uses

Tetrabenazine exerts its therapeutic effects by inhibiting the vesicular monoamine transporter VMAT2 in the brain and by inhibiting both pre-synaptic and postsynaptic dopamine receptors.

The novel dihydrotetrabenazine isomers of the invention are also inhibitors of VMAT2, with Isomers C and B producing the greatest degree of inhibition. Like tetrabenazine, the compounds of the invention have only a low affinity for VMAT1, the VMAT isoform found in peripheral tissues and some endocrine cells, thereby indicating that they should not produce the side effects associated with reserpine. Compounds C and B also exhibit no inhibitory activity against catechol O-methyl transferase (COMT), monoamine oxidase isoforms A and B, and 5-hydroxytryptamine isoforms 1d and 1b.

Surprisingly, isomers C and B also show a remarkable separation of VAMT2 and dopamine receptor activity in that although they are highly active in binding VMAT2, both compounds exhibit only weak or non-existent dopamine receptor binding activity and lack Dopamine Transporter (DAT) binding activity. In fact, none of the isomers exhibit significant DAT binding activity. This suggests that the compounds may lack the dopaminergic side effects produced by tetrabenazine. Isomers C and B are also either weakly active or inactive as inhibitors of the adrenergic receptors and this suggests that the compounds may lack the adrenergic side effects often encountered with tetrabenazine. In fact, in locomotor studies carried out on rats, tetrabenazine exhibited a dose related sedative effect, whereas no sedative effects were observed following administration of the dihydrotetrabenazine isomers B and C of the invention.

Furthermore, both Isomer C and Isomer B are potent inhibitors of the serotonin transporter protein SERT. Inhibition of SERT is one mechanism by which antidepressants such as fluoxetine (Prozac®) exert their therapeutic effects. Therefore, the ability of Isomers C and B to inhibit SERT indicates that these isomers may act as antidepressants, in marked contrast to tetrabenazine for which depression is a well recognised side effect.

Isomer B has been tested in a transgenic mouse model of Huntington's disease and has been shown to arrest the progression of a number of symptoms of Huntington's disease, including involuntary movements such as involuntary chorea, tremors and twitches, and deterioration in gait. On the basis of the studies carried out to date, it is envisaged that the cis-dihydrotetrabenazine compounds of the invention will therefore be useful in the treatment of Huntington's disease, and in particular for arresting or slowing down the progression of the disease, or for use in a prophylactic manner to prevent development of the disease.

The compounds will generally be administered to a subject in need of such administration, for example a human or animal patient, preferably a human.

The compounds will typically be administered in amounts that are therapeutically or prophylactically useful and which generally are non-toxic. However, in certain situations, the benefits of administering a dihydrotetrabenazine compound of the invention may outweigh the disadvantages of any toxic effects or side effects, in which case it may be considered desirable to administer compounds in amounts that are associated with a degree of toxicity.

A typical daily dose of the compound can be up to 1000 mg per day, for example in the range from 0.01 milligrams to 10 milligrams per kilogram of body weight, more usually from 0.025 milligrams to 5 milligrams per kilogram of body weight, for example up to 3 milligrams per kilogram of bodyweight, and more typically 0.15 milligrams to 5 milligrams per kilogram of bodyweight although higher or lower doses may be administered where required.

By way of example, an initial starting dose of 12.5 mg may be administered 2 to 3 times a day. The dosage can be increased by 12,5 mg a day every 3 to 5 days until the maximal tolerated and effective dose is reached for the individual as determined by the physician. Ultimately, the quantity of compound administered will be commensurate with the nature of the disease or physiological condition being treated and the therapeutic benefits and the presence or absence of side effects produced by a given dosage regimen, and will be at the discretion of the physician.

### Pharmaceutical Formulations

The dihydrotetrabenazine compounds are typically administered in the form of pharmaceutical compositions.

The pharmaceutical compositions can be in any form suitable for oral, parenteral, topical, intranasal, intrabronchial, ophthalmic, otic, rectal, intra-vaginal, or transdermal administration. Where the compositions are intended for parenteral administration, they can be formulated for intravenous, intramuscular, intraperitoneal, subcutaneous administration or for direct delivery into a target organ or tissue by injection, infusion or other means of delivery.

Pharmaceutical dosage forms suitable for oral administration include tablets, capsules; caplets, pills, lozenges, syrups, solutions, sprays, powders, granules, elixirs and suspensions, sublingual tablets, sprays, wafers or patches and buccal patches.

Pharmaceutical compositions containing the dihydrotetrabenazine compounds of the invention can be formulated in accordance with known techniques, see for example, Remington's Pharmaceutical Sciences, Mack Publishing Company, Easton, PA, USA.

Thus, tablet compositions can contain a unit dosage of active compound together with an inert diluent or carrier such as a sugar or sugar alcohol, e.g.; lactose, sucrose, sorbitol or mannitol; and/or a non-sugar derived diluent such as sodium carbonate, calcium phosphate, talc, calcium carbonate, or a cellulose or derivative thereof such as methyl cellulose, ethyl cellulose, hydroxypropyl methyl cellulose, and starches such as corn starch. Tablets may also contain such standard ingredients as binding and granulating agents such as polyvinylpyrrolidone, disintegrants (e.g. swellable crosslinked polymers such as crosslinked carboxymethylcellulose), lubricating agents (e.g. stearates), preservatives (e.g. parabens), antioxidants (e.g. BHT), buffering agents (for example phosphate or citrate buffers), and effervescent agents such as citrate/bicarbonate mixtures. Such excipients are well known and do not need to be discussed in detail here.

Capsule formulations may be of the hard gelatin or soft gelatin variety and can contain the active component in solid, semi-solid, or liquid form. Gelatin capsules can be formed from animal gelatin or synthetic or plant derived equivalents thereof.

The solid dosage forms (e,g.; tablets, capsules etc.) can be coated or un-coated, but typically have a coating, for example a protective film coating (e.g, a wax or varnish) or a release controlling coating. The coating (e.g. a Eudragit^{™} type polymer) can be designed to release the active component at a desired location within the gastro-intestinal tract. Thus, the coating can be selected so as to degrade under certain pH conditions within the gastrointestinal tract, thereby selectively release the compound in the stomach or in the ileum or duodenum.

Instead of, or in addition to, a coating, the drug can be presented in a solid matrix comprising a release controlling agent, for example a release delaying agent which may be adapted to selectively release the compound under conditions of varying acidity or alkalinity in the gastrointestinal tract. Alternatively, the matrix material or release retarding coating can take the form of an erodible polymer (e.g. a maleic anhydride polymer) which is substantially continuously eroded as the dosage form passes through the gastrointestinal tract.

Compositions for topical use include ointments, creams, sprays, patches, gels, liquid drops and inserts (for example intraocular inserts). Such compositions can be formulated in accordance with known methods.

Compositions for parenteral administration are typically presented as sterile aqueous or oily solutions or fine suspensions, or may be provided in finely divided sterile powder form for making up extemporaneously with sterile water for injection.

Examples of formulations for rectal or intra-vaginal administration include pessaries and suppositories which may be, for example, formed from a shaped mouldable or waxy material containing the active compound.

Compositions for administration by inhalation may take the form of inhalable powder compositions or liquid or powder sprays, and can be administrated in standard form using powder inhaler devices or aerosol dispensing devices. Such devices are well knows. For administration by inhalation, the powdered formulations typically comprise the active compound together with an inert solid powdered diluent such as lactose.

The compounds of the inventions will generally be presented in unit dosage for and, as such, will typically contain sufficient compound to provide a desired level of biological activity. For example, a formulation intended for oral administration may contain from 2 milligrams to 200 milligrams of active ingredient, more usually from 10 milligrams to 100 milligrams, for example, 12.5 milligrams, 25 milligrams and 50 milligrams.

The active compound will be administered to a patient in need thereof (for example a human or animal patient) in an amount sufficient to achieve the desired therapeutic effect.

### EXAMPLES

The following non-limiting examples illustrate the synthesis and properties of the dihydrotetrabenazine compounds of the invention.

### EXAMPLE 1

### Preparation of 2S,3S,11 b/R and 2R,3R,11bS Isomers of Dihydrotetrabenazine

### 1 A. Reduction of RR/SS Tetrabenazine

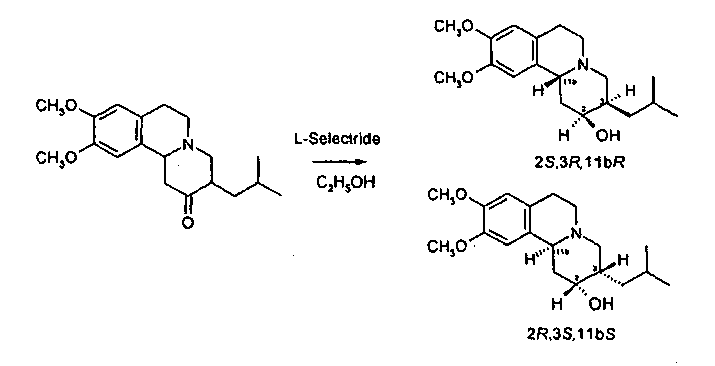

1M L-Selectride^{®} in tetrahydrofuran (135 ml, 135 mmol, 2.87 eq.) was added slowly over 30 minutes to a stirred solution of tetrabenazine *RR*/*SS* racemate (15g, 47 mmol) in ethanol (75 ml) and tetrahydrofuran (75 ml) at 0 °C, After addition was complete the mixture was stirred at 0 °C for 30 minutes and then allowed to warm to room temperature.

The mixture was poured onto crushed ice (300 g) and water (100 ml) added. The solution was extracted with diethyl ether (2 x 200 ml) and the combined ethereal extracts washed with water (100 ml) and partly dried over anhydrous potassium carbonate. Drying was completed using anhydrous magnesium sulphate and, after filtration, the solvent was removed at reduced pressure (shielded from the light, bath temperature <20 °C) to afford a pale yellow solid.

The solid was slurried with petroleum ether (30-40 °C) and filtered to afford a white powdery solid (12 g, 80%).

### 1B. Dehydration of reduced Tetrabenazine

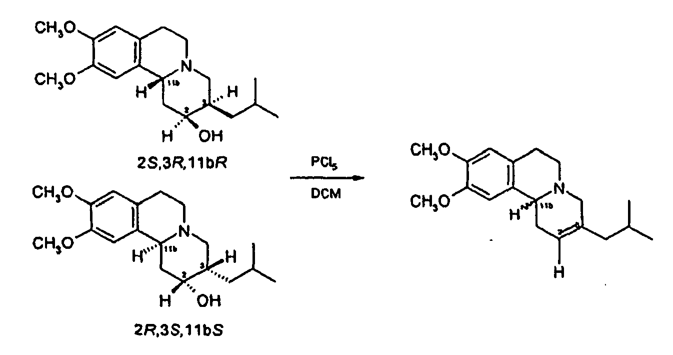

Phosphorous pentachloride (32.8 g, 157.5 mmol, 2.5 eq) was added in portions over 30 minutes to a stirred solution of the reduced tetrabenazine product from Example 1A (20 g, 62.7 mmol) in dichloromethane (200 ml) at 0 °C. After the addition was complete, the reaction mixture was stirred at 0 °C for a further 30 minutes and the solution poured slowly into 2M aqueous sodium carbonate solution containing crushed ice (0 °C). Once the initial acid gas evolution had ceased the mixture was basified (ca. pH 12) using solid sodium carbonate.

The alkaline solution was extracted using ethyl acetate (800 ml) and the combined organic extracts dried over anhydrous magnesium sulphate. After filtration the solvent was removed at reduced pressure to afford a brown oil, which was purified by column chromatography (silica, ethyl acetate) to afford the semi-pure alkene as a yellow solid (10.87 g, 58%).

### 1C. Hydration of the Crude Alkene from Example 1B

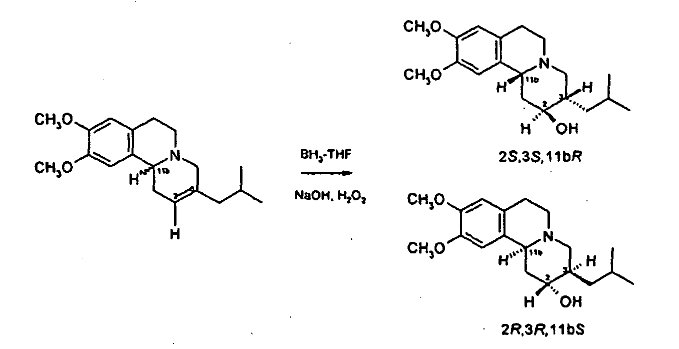

A solution of the crude alkene (10.87 g, 36.11 mmol) from Example 1B in dry THF (52 ml) at room temperature was treated with 1M borane-THF (155.6 ml, 155.6 mmol), 4.30 eq) added in a dropwise manner. The reaction was stirred for 2 hours, water (20 ml) was added and the solution basified to pH 12 with 30% aqueous sodium hydroxide solution.

Aqueous 30% hydrogen peroxide solution (30 ml) was added to the stirred alkaline reaction mixture and the solution was heated to reflux for 1 hour before being allowed to cool. Water (100 ml) was added and the mixture extracted with ethyl acetate (3 x 250 ml). The organic extracts were combined and dried over anhydrous magnesium sulphate and after filtration the solvent was removed at reduced pressure to afford a yellow oil (9 g).

The oil was purified using preparative HPLC (Column: Lichrospher Si60, 5 µm, 250 x 21.20 mm, mobile phase: hexane : ethanol; dichloromethane (85:15:5); UV 254 nm, flow: 10 ml min⁻¹) at 350 mg per injection followed by concentration of the fractions of interest under vacuum. The product oil was then dissolved in ether and concentrated once more under vacuum to give the dihydrotetrabenazine racemate shown above as a yellow foam (5.76 g, 50%).

### 1D. Preparation of Mosher's ester derivatives

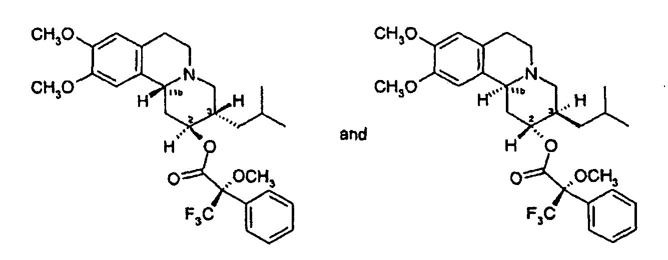

R-(+)-α-methoxy-α-trifluoromethylphenyl acetic acid (5 g, 21.35 mmol), oxalyl chloride (2.02 ml) and DMF (0.16 ml) were added to anhydrous dichloromethane (50 ml) and the solution was stirred at room temperature for 45 minutes. The solution was concentrated under reduced pressure and the residue was taken up in anhydrous dichloromethane (50 ml) once more. The resulting solution was cooled using an ice-water bath and dimethylaminopyridine (3.83 g, 31.34 mmol) was added followed by a pre-dried solution (over 4Å sieves) in anhydrous dichloromethane of the solid product of Example 1C (5 g, 15.6 mmol). After stirring at room temperature for 45 minutes, water (234 ml) was added and the mixture extracted with ether (2 x 200 ml). The ether extract was dried over anhydrous magnesium sulphate, passed through a pad of silica and the product elated using ether.

The collected ether eluate was concentrated under reduced pressure to afford an oil which was purified using column chromatography (silica, hexane : ether (10:1)). Evaporation of the collected column fractions of interest and removal of the solvent at reduced pressure gave a solid which was further purified using column chromatography (silica, hexane : ethyl acetate (1:1)) to give three main components which were partially resolved into Mosher's ester peaks 1 and 2.

Preparative HPLC of the three components (column: 2 x Lichrospher Si60, 5 µm, 250 x 21.20 mm, mobile phase: hexane: isopropanol (97:3), UV 254 nm; flow: 10 ml min⁻¹) at 300 mg loading followed by concentration of the fractions of interest under vacuum gave the pure Mosher's ester derivatives
Peak 1 (3.89 g, 46.5%)
Peak 2 (2.78 g, 33%)

The fractions corresponding to the two peaks were subjected to hydrolysis to liberate the individual dihydrotetrabenazine isomers identified and characterised as Isomers A and B. Isomers A and B are each believed to have one of the following structures

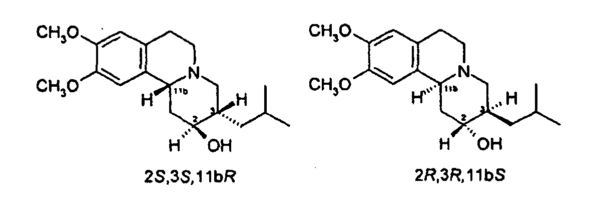

More specifically, Isomer B is believed to have the 2S, *3S,* 11b*R* absolute configuration on the basis of the X-ray crystallography experiments described in Example 4 below.

### 1E. Hydrolysis of Peak 1 to give Isomer A

Aqueous 20% sodium hydroxide solution (87.5 ml) was added to a solution of Mosher's ester peak 1 (3.89 g, 7.27 mmol) in methanol (260 ml) and the mixture stirred and heated to reflux for 150 minutes. After cooling to room temperature water (200 ml) was added and the solution extracted with ether (600 ml), dried over anhydrous magnesium sulphate and after filtration, concentrated under reduced pressure.

The residue was dissolved using ethyl acetate (200 ml), the solution washed with water (2 x 50 ml), the organic phase dried over anhydrous magnesium sulphate and after filtration, concentrated under reduced pressure to give a yellow foam. This material was purified by column chromatography (silica, gradient elution of ethyl acetate : hexane (1:1) to ethyl acetate). The fractions of interest were combined and the solvent removed at reduced pressure. The residue was taken up in ether and the solvent removed at reduced pressure once more to give Isomer A as an off-white foam (1.1 g, 47%).

Isomer A, which is believed to have the 2*R*,3*R*,11b*S* configuration (the absolute stereochemistry was not determined), was characterized by ¹H-NMR, ¹³C-NMR.
IR, mass spectrometry, chiral HPLC and ORD. The IR, NMR and MS data for isomer A are set out in Table 1 and the Chiral HPLC and ORD data are set out in Table 3.

### 1F. Hydrolysis of Peak 2 to give Isomer B

Aqueous 20% sodium hydroxide solution (62.5 ml) was added to a solution of Mosher's ester peak 2 (2.78 g, 5.19 mmol) in methanol (185 ml) and the mixture stirred and heated to reflux for 150 minutes. After cooling to room temperature water (142 ml) was added and the solution extracted with ether (440 ml), dried over anhydrous magnesium sulphate and after filtration, concentrated under reduced pressure.

The residue was dissolved using ethyl acetate (200 ml), the solution washed with water (2 x 50 ml), the organic phase dried over anhydrous magnesium sulphate and after filtration, concentrated under reduced pressure. Petroleum ether (30-40 °C) was added to the residue and the solution concentrated under vacuum once more to give Isomer B as a white foam (1.34 g, 81%).

Isomer B, which is believed to have the 2*S*,3*S*,11b*R* configuration, was characterized by ¹H-NMR, ¹³C-NMR, IR, mass spectrometry, chiral HPLC, ORD and X-ray crystallography, The IR, NMR and MS data for Isomer B are set out in Table 1 and the Chiral HPLC and ORD data are set out in Table 3. The X-ray crystallography data are set out in Example 4.

### EXAMPLE 2

### Preparation of 2R, 3S,11bR and 2S,3R,11bS Isomers of Dihydrotetrabenazine

### 2A. Preparation of 2,3-Dehydrotetrabenazine

A solution containing a racemic mixture (15 g, 47 mmol) of *RR* and *SS* tetrabenazine enantiomers in tetrahydrofuran was subjected to reduction with L-Selectride^{®} by the method of Example 1A to give a mixture of the 2S,3R,1 IbR and 2*R*,3*S*,11b*S* enantiomers of dihydrotetrabenazine as a white powdery solid (12 g, 80%). The partially purified dihydrotetrabenazine was then dehydrated using PCl₅ according to the method of Example 1B to give a semi-pure mixture of 11b*R* and 11 bS isomers of 2,3-dehydrotetrabenazine (the 11b*R* enantiomer of which is shown below) as a yellow solid (12.92 g, 68%).

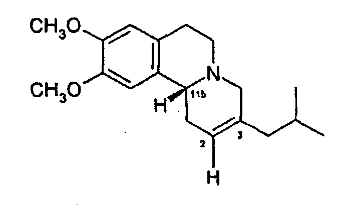

### 2B. Epoxidation of the Crude Alkene from Example 2A

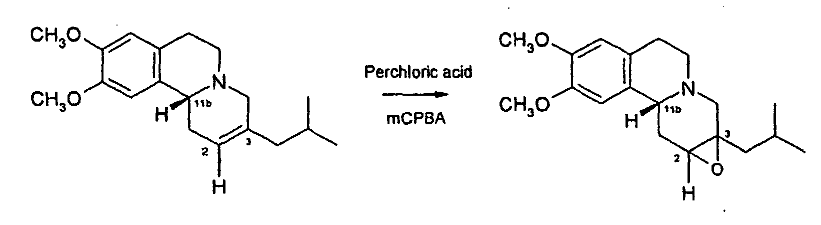

To a stirred solution of the crude alkene from Example 2A (12,92 g, 42.9 mmol) in methanol (215 ml) was added a solution of 70% perchloric acid (3.70 ml, 43 mmol) in methanol (215 ml). 77% 3-Chloroperoxybenzoic acid (15.50 g, 65 mmol) was added to the reaction and the resulting mixture was stirred for 18 hours at room temperature protected from light.

The reaction mixture was poured into saturated aqueous sodium sulphite solution. (200 ml) and water (200 ml) added. Chloroform (300 ml) was added to the resulting emulsion and the mixture basified with saturated aqueous sodium bicarbonate (400 ml).

The organic layer was collected and the aqueous phase washed with additional chloroform (2 x 150 ml). The combined chloroform layers were dried over anhydrous magnesium sulphate and after filtration the solvent was removed at reduced pressure to give a brown oil (14.35 g, yield > 100% probable solvent remain in product). This material was used without further purification.

### 2C. Reductive Ring Opening of the Epoxide from 2B

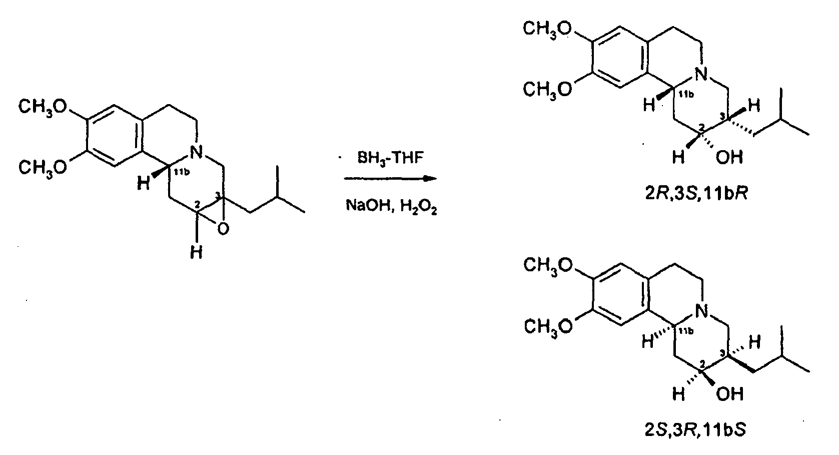

A stirred solution of the crude epoxide from Example 2B (14.35 g, 42,9 mmol, assuming 100% yield) in dry THF (80 ml) was treated slowly with 1M borane/THF (184.6 ml, 184.6 mmol) over 15 minutes. The reaction was stirred for two hours, water (65 ml) was added and the solution heated with stirring to reflux for 30 minutes.

After cooling, 30% sodium hydroxide solution (97 ml) was added to the reaction mixture followed by 30% hydrogen peroxide solution (48.6 ml) and the reaction was stirred and heated to reflux for an additional 1 hour.

The cooled reaction mixture was extracted with ethyl acetate (500 ml) dried over anhydrous magnesium sulphate and after filtration the solvent was removed at reduced pressure to give an oil. Hexane (230 ml) was added to the oil and the solution re-concentrated under reduced pressure.

The oily residue was purified by column chromatography (silica, ethyl acetate), The fractions of interest were combined and the solvent removed under reduced pressure. The residue was purified once more using column chromatography (silica, gradient, hexane to ether). The fractions of interest were combined and the solvents evaporated at reduced pressure to give a pale yellow solid (5.18 g, 38%),

### 2D. Preparation of Mosher's ester derivatives of the 2R,3S,11bR and 2S,3R,11bS Isomers of Dihydrotetrabenazine

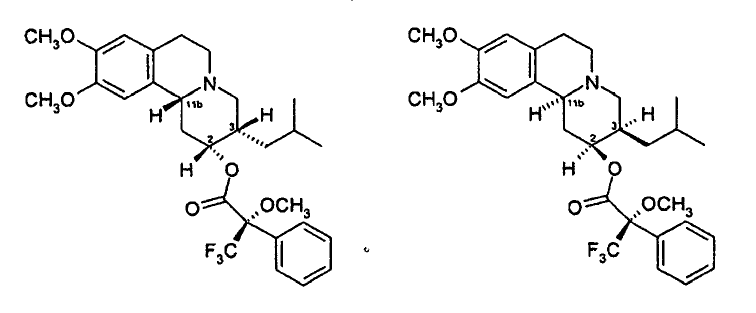

R-(+)-α-methoxy-α-trifluoromethylphenyl acetic acid (4.68 g, 19.98 mmol)), oxalyl chloride (1,90 ml) and DMF (0.13 ml) were added to anhydrous dichloromethane (46 ml) and the solution stirred at room temperature for 45 minutes. The solution was concentrated under reduced pressure and the residue was taken up in anhydrous dichloromethane (40 ml) once more. The resulting solution was cooled using an ice-water bath and dimethylaminopyridine (3.65 g, 29.87 mmol) was added followed by a pre-dried solution (over 4Å sieves) in anhydrous dichloromethane (20 ml) of the solid product of Example 2C (4.68 g, 14.6 mmol). After stirring at room temperature for 45 minutes, water (234 ml) was added and the mixture extracted with ether (2 x 200 ml). The ether extract was dried over anhydrous magnesium sulphate, passed through a pad of silica and the product eluted using ether.

The collected ether eluate was concentrated under reduced pressure to afford an oil which was purified using column chromatography (silica, hexane ; ether (1:1)).

Evaporation of the collected column fractions of interest and removal of the solvent at reduced pressure gave a pink solid (6.53 g)

Preparative HPLC of the solid (Column: 2 x Lichrospher Si60, 5 µm, 250 x 21.20 mm; mobile phase hexane : isopropanol (97:3); UV 254 nm; flow: 10 ml min⁻¹) at 100 mg loading followed by concentration of the fractions of interest under vacuum gave a solid which was slurried with petroleum ether (30-40 °C) and collected by Nitration to give the pure Mosher's ester derivatives
Peak 1 (2.37 g, 30%)
Peak 2 (2.42 g, 30%)

The fractions corresponding to the two peaks were subjected to hydrolysis to liberate the individual dihydrotetrabenazine isomers identified and characterised as Isomers C and D. Isomers C and D are each believed to have one of the following structures

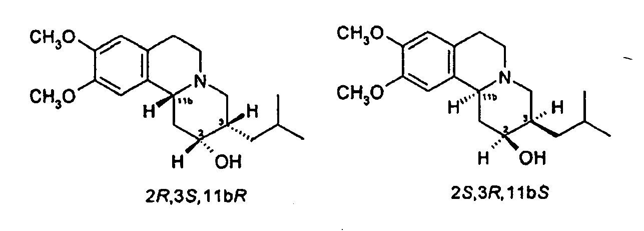

### 2F. Hydrolysis of Peak 1 to give Isomer C

20% aqueous sodium hydroxide solution (53 ml) was added to a stirred solution of Mosher's ester peak 1 (2.37 g, 4.43 mmol) in methanol (158 ml) and the mixture stirred at reflux for 150 minutes. After cooling water (88 ml) was added to the reaction mixture and the resulting solution extracted with ether (576 ml). The organic extract was dried over anhydrous magnesium sulphate and after filtration the solvent removed at reduced pressure. Ethyl acetate (200 ml) was added to the residue and the solution washed with water (2 x 50 ml). The organic solution was dried over anhydrous magnesium sulphate and after filtration the solvent removed at reduced pressure.

This residue was treated with petroleum ether (30-40 °C) and the resulting suspended solid collected by filtration. The filtrate was concentrated at reduced pressure and the second batch of suspended solid was collected by filtration. Both collected solids were combined and dried under reduced pressure to give Isomer C (1.0g, 70%).

Isomer C, which is believed to have either the 2*R*,3*S*,11b*R* or 2*S*,3*R*, 11b*S* configuration (the absolute stereochemistry was not determined), was characterized by ¹H-NMR, ¹³C-NMR, IR, mass spectrometry, chiral HPLC and ORD. The IR, NMR and MS data for Isomer C are set out in Table 2 and the Chiral HPLC and ORD data are set out in Table 4.

### 2G, Hydrolysis of Peak 2 to give Isomer D

20% aqueous sodium hydroxide solution (53 ml) was added to a stirred solution of Mosher's ester peak 2 (2.42 g, 4.52 mmol) in methanol (158 ml) and the mixture stirred at reflux for 150 minutes. After cooling water (88 ml) was added to the reaction mixture and the resulting solution extracted with ether (576 ml). The organic extract was dried over anhydrous magnesium sulphate and after filtration the solvent removed at reduced pressure. Ethyl acetate (200 ml) was added to the residue and the solution washed with water (2 x 50 ml). The organic solution was dried over anhydrous magnesium sulphate and after filtration the solvent removed at reduced pressure.

This residue was treated with petroleum ether (30-40 °C) and the resulting suspended orange solid collected by filtration. The solid was dissolved in ethyl acetate : hexane (15:85) and purified by column chromatography (silica, gradient ethyl acetate : hexane (15:85) to ethyl acetate). The fractions of interest were combined and the solvent removed at reduced pressure. The residue was slurried with petroleum ether (30-40 °C) and the resulting suspension collected by filtration, The collected solid was dried under reduced pressure to give Isomer D as a white solid (0.93 g, 64%).

Isomer D, which is believed to have either the 2*R*,3*S*,11b*R* or 2*S*,3*R*,11b*S* configuration (the absolute stereochemistry was not determined), was characterized by ¹H-NMR, ¹³C-NMR, IR, mass spectrometry, chiral HPLC and ORD. The IR, NMR and MS data for Isomer D are set out in Table 2 and the Chiral HPLC and ORD data are set out in Table 4.

In Tables 1 and 2, the infra red spectra were determined using the KBr disc method. The ¹H NMR spectra were carried out on solutions in deuterated chloroform using a Varian Gemini NMR spectrometer (200 MHz.). The ¹³C NMR spectra were carried out on solutions in deuterated chloroform using a Varian Gemini NMR spectrometer (50MHz). The mass spectra were obtained using a Micromass Platform II (ES⁺ conditions) spectrometer. In Tables 3 and 4, the Optical Rotatory Dispersion figures were obtained using an Optical Activity Polar 2001 instrument in methanol solution at 24°C. The HPLC retention time measurements were carried out using an HP1050 HPLC chromatograph with UV detection.

### Tables 1 and 2 - Spectroscopic Data

| Table 1 | | | | |
|---|---|---|---|---|
| Dihydrotetrabenazine isomer | ¹H-NMR spectrum (CDCl₃) | ¹³C-NMR spectrum (CDCl₃) | IR Spectrum (KBr solid) | Mass Spectrum (ES⁺) |
| Isomers A and B | 6.67δ 1H (s); 6.57 δ1H (s); 3.84 δ 6H (s); 3.55 δ 1H (br. d); 3.08 δ1H (m); 2.79 δ 2H (m); 2.55 δ 3H (m); 2.17 δ 1H (m); 1.72 δ 6H (m); 1.02 δ 1H (m); 0.88 δ 6H (t) | 147.7δ; 147.6δ; 130.5δ; 127.6δ; 112.1δ; 108.4δ; 70.5δ; 57.5 δ; 56.3δ; 56.3 δ; 54.8 δ; 53.2 δ; 40.4 δ; 40.1δ; 36.0 δ; 28.8 δ; 26.2 δ; 23.7δ; 22.9 δ | 2950 cm⁻¹; 2928 cm⁻¹; 2868 cm⁻¹; 2834 cm⁻¹; 1610 cm⁻¹; 1511 cm⁻¹ ; 1464 cm⁻¹ 1364 cm⁻¹; 1324 cm⁻¹; 1258 cm⁻¹; 1223 cm⁻¹; 1208 cm⁻¹; 1144 cm⁻¹; 1045 cm⁻¹; 1006 cm⁻¹; 870 cm⁻¹; 785 cm⁻¹; 764 cm⁻¹ | MH⁺ 320 |
| 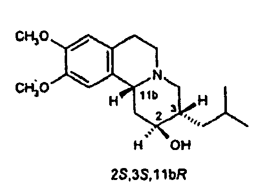 | | | | |
| OR | | | | |
| 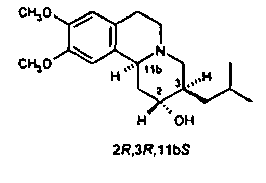 | | | | |

| Table 2 | | | | |
|---|---|---|---|---|
| Dihydrotetrabenazine isomer | ¹H-NMR spectrum (CDCl₃) | ¹³C-NMR spectrum (CDCl₃), | IR Spectrum (KBr solid) | Mass Spectrum (ES⁺) |
| Isomers C and D | 6.68 δ 1H (s); 6.58 δ 1H (s); 3.92 δ 1H (m); 3.84 δ 6H (s); 3.15 δ 1H (m); 2.87 δ 3H (m); 2.43 δ 4H (m); 1.81 δ 1H (m); 1.64 δ 4H (m); 1.216 δ 1H (m); 0.94 δ 3H (d); 0.89 δ 3H (d) | 147.8 δ; 147.7 δ; 130.4 δ; 127.2 δ; 112.0 δ; 108.3 δ; 72.4 δ; 61.2 δ; 58.3 δ; 56.5 δ; 56.3 δ; 52.7 δ; 38.6 δ; 36.7 δ; 34.4δ; 29.6δ; 26.5 δ; 24.4 δ; 22.5 δ | 3370 cm⁻¹; 2950 cm⁻¹; 2929 cm⁻¹; 1611 cm⁻¹; 1512 cm⁻¹; 1463 cm⁻¹ 1362 cm⁻¹; 1334 cm⁻¹; 1259 cm⁻¹; 1227 cm⁻¹; 1148 cm⁻²; 1063 cm⁻¹; 1024 cm⁻¹; 855 cm⁻¹; 766 cm⁻¹ | MH⁺320 |
| 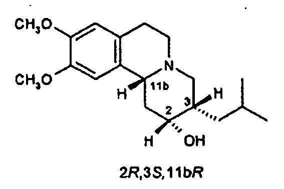 | | | | |
| OR | | | | |
| 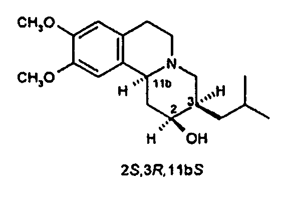 | | | | |

### Tables 3 and 4 - Chromatographs and ORD Data

| Table 3 | | | |
|---|---|---|---|
| Dihydrotetrabenazine isomer | Chiral HPLC Methods and Retention Times | | ORD (MeOH, 21°C) |
| Isomers A and B | Column: | | Isomer A |
| 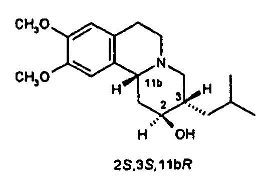 | Chirex (S)-VAL, (R)-NEA, 250 x 4,6 mm | | [α_{D}]-114.6° |
| | Mobile phase: ethanol (36:62:2) | Hexane : 1,2-dichloroethane : | |
| | Flow: | 1.0 ml min⁻¹ | Isomers B |
| | UV: | 234 nm | [α_{D}] +123° |
| OR | | | |
| 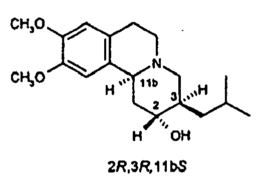 | Retention times: | | |
| | isomer A | 14.6 min | |
| | Isomer | 15.3 min | |

| Table 4 | | | |
|---|---|---|---|
| Isomers C and D | Column: | | Isomer C |
| 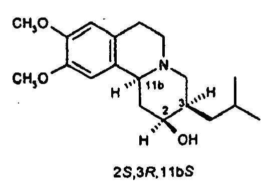 | Chirex (S)-VAL, | (R)-NEA, 250 x 4.6mtn | [α_{D}] +150.9° |
| | Mobile phase: | Hexane : ethanol (92:8) | |
| | Flow: | 1.0 m] min⁻¹ | Isomer D |
| | UV: | 254 nm | [α_{D}] -145.7° |
| OR | | | |
| 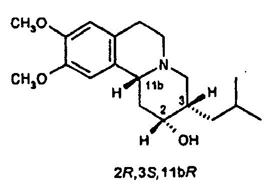 | Retention times: | | |
| | Isomer C | 20.3 min | |
| | Isomer D | 19.4 min | |

### EXAMPLE 3

### Alternative Method of Preparation of Isomer B and Preparation of Mesylate salt 3A. Reduction of RR/SS Tetrabenazine

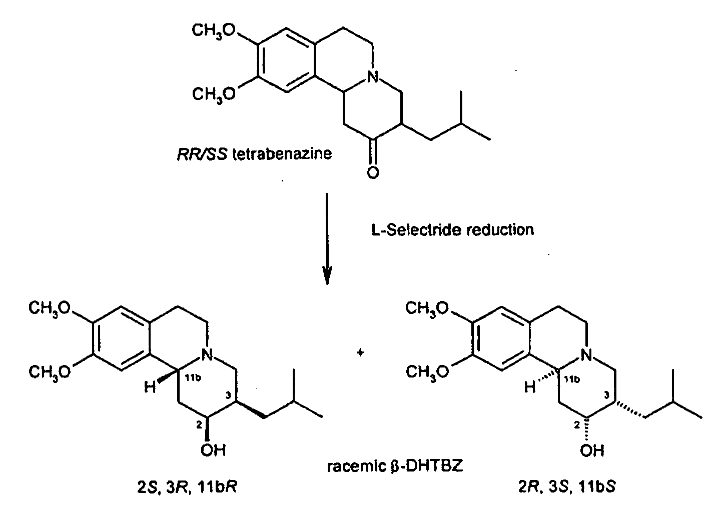

1M L-Selectride^{®} in tetrahydrofuran (52 ml, 52 mmol, 1.1 eq) was added slowly over 30 minutes to a cooled (ice bath), stirred solution of tetrabenazine racemate (15 g, 47 mmol) in tetrahydrofuran (56 ml). After the addition was complete, the mixture was allowed to warm to room temperature and stirred for a further six hours. TLC analysis (silica, ethyl acetate) showed only very minor amounts of starting material remained.

The mixture was poured on to a stirred mixture of crushed ice (112 g), water (56 ml) and glacial acetic acid (12.2 g). The resulting yellow solution was washed with ether (2 x 50 ml) and basified by the slow addition of solid sodium carbonate (ca. 13 g). Pet-ether (30-40 °C) (56 ml) was added to the mixture with stirring and the crude β-DHTBZ was collected as a white solid by filtration.

The crude solid was dissolved in dichloromethane (ca. 150 ml) and the resulting solution washed with water (40 ml), dried using anhydrous magnesium sulphate, filtered and concentrated at reduced pressure to ca. 40 ml. A thick suspension of white solid was formed. Pet-ether (30-40 °C) (56 ml) was added and the suspension was stirred for fifteen minutes at laboratory temperature. The product was collected by filtration and washed on the filter until snow-white using pet-ether (30-40°C) (40 to 60 ml) before air-drying at room temperature to yield β-DHTBZ (10.1 g, 67%) as a white solid. TLC analysis (silica, ethyl acetate) showed only one component.

### 3B. Preparation and Fractional Crystallisation of the Camphorsulphonic acid Salt of Racemic β-DHTBZ

The product of Example 3A and 1 equivalent of (S)-(+)-Camphor-10-sulphonic acid were dissolved with heating in the minimum amount of methanol. The resulting solution was allowed to cool and then diluted slowly with ether until formation of the resulting solid precipitation was complete. The resulting white crystalline solid was collected by filtration and washed with ether before drying.

The camphorsulphonic acid salt of (10 g) was dissolved in a mixture of hot absolute ethanol (170 ml) and ethanol (30 ml). The resulting solution was stirred and allowed to cool. After two hours the precipitate formed was collected by filtration as a white crystalline solid (2.9 g). A sample of the crystalline material was shaken in a separating funnel with excess saturated aqueous sodium carbonate and dichloromethane. The organic phase was separated, dried over anhydrous magnesium sulphate, filtered and concentrated at reduced pressure, The residue was triturated using pet-ether (30"40 °C) and the organic solution concentrated once more. Chiral HPLC analysis of the salt using a Chirex (S)-VAL and (R)-NEA 250 x 4.6 mm column, and a hexane : ethanol (98:2) eluent at a flow rate of 1 ml/minute showed showed that the isolated β-OHTBZ was enriched in one enantiomer (e.e. ca. 80%).

The enriched camphorsulphonic acid salt (14 g) was dissolved in hot absolute ethanol (140 ml) and propan-2-ol (420 ml) was added. The resulting solution was stirred and a precipitate began to form within one minute. The mixture was allowed to cool to room temperature and stirred for one hour. The precipitate formed was collected by filtration, washed with ether and dried to give a white crystalline solid (12 g).

The crystalline material was shaken in a separating funnel with excess saturated aqueous sodium carbonate and dichloromethane. The organic phase was separated, dried over anhydrous magnesium sulphate, filtered and concentrated at reduced pressure. The residue was triturated using pet-ether (30-40 °C) and the organic solution concentrated once more to yield (after drying in vacuo.) (+)-β-DHTBZ (6.6 g, ORD +107.8°). The isolated enantiomer has e.e. >97%.

### 3C. Preparation of Isomer B

A solution of phosphorus pentachloride (4,5 g, 21.6 mmol, 1.05 eq) in dichloromethane (55 ml) was added steadily over ten minutes to a stirred, cooled (ice-water bath) solution of the product of Example 3B (6.6 g, 20.6 mmol) in dichloromethane (90 ml). When the addition was complete, the resulting yellow solution was stirred for a further ten minutes before pouring on to a rapidly stirred mixture of sodium carbonate (15 g) in water (90 ml) and crushed ice (90 g). The mixture was stirred for a further 10 minutes and transferred to a separating funnel.

Once the phases had separated, the brown dichloromethane layer was removed, dried over anhydrous magnesium sulphate, filtered and concentrated at reduced pressure to give the crude alkene intermediate as brown oil (ca. 6.7 g). TLC analysis (silica, ethyl acetate) showed that no (+)-β-DHTBZ remained in the crude product.

The crude alkene was taken up (dry nitrogen atmosphere) in anhydrous tetrahydrofuran (40 ml) and a solution of borane in THF (1 M solution, 2.5 eq, 52 ml) was added with stirring over fifteen minutes. The reaction mixture was then stirred at room temperature for two hours. TLC analysis (silica, ethyl acetate) showed that no alkene intermediate remained in the reaction mixture.

A solution of sodium hydroxide (3.7 g) in water (10 ml) was added to the stirring reaction mixture, followed by an aqueous solution of hydrogen peroxide (50%, ca. 7 ml) and the two-phase mixture formed was stirred at reflux for one hours. TLC analysis of the organic phase at this time (silica, ethyl acetate) showed the appearance of a product with Rf as expected for Isomer B. A characteristic nonpolar component was also seen.

The reaction mixture was allowed to cool to room temperature and was poured into a separating funnel. The upper organic layer was removed and concentrated under reduced pressure to remove the majority of THF. The residue was taken up in ether (stabilised (BHT), 75 ml), washed with water (40 ml), dried over anhydrous magnesium sulphate, filtered and concentrated under reduced pressure to give a pale yellow oil (8.1 g).

The yellow oil was purified using column chromatography (silica, ethyl acetate: hexane (80:20), increasing to 100% ethyl acetate) and the desired column fractions collected, combined and concentrated at reduced pressure to give a pale oil which was treated with ether (stabilised, 18 ml) and concentrated at reduced pressure to give Isomer B as a pale yellow solid foam (2.2 g).

Chiral HPLC using the conditions set out in Example 3B confirmed that Isomer B had been produced in an enantiomeric excess (e.e.) of greater than 97%.

The optical rotation was measured using a Bellingham Stanley ADP220 polarimeter and gave an [α_{D}] of+123.5°.

### 3D. Preparation of the Mesylate salt of Isomer B

The methanesulphonate salt of Isomer B was prepared by dissolving a mixture of 1 equivalent of Isomer B from Example 3C and 1 equivalent of methane sulphonic acid in the minimum amount of ethanol and then adding diethyl ether. The resulting white precipitate that formed was collected by filtration and dried *in vacuo* to give the mesylate salt in a yield of ca. 85% and a purity (by HPLC) of ca, 96%.

### EXAMPLE 4

### X-Ray Crystallographic Studies on Isomer B

The (S)-(+)-Camphor-10-sulpbonic acid salt of Isomer B was prepared and a single crystal was subjected to X-ray crystallographic studies under the following conditions:

Diffractometer: Nonius KappaCCD area detector (t/i scans and OJ scans to fill asymmetric unit).

Cell determination: DirAx (Duisenberg, A.J.M.( 1992). J. Appl. Cryst. 25, 92-96.)

Data collection: Collect (Collect: Data collection software, R. Hooft, Nonius B. V, 1998)

Data reduction and cell refinement: Demo (Z. Otwinowski & W. Minor, Methods in Enzymology (1997) Vol. 276: Macromolecular Crystallography, part A, pp. 307-326; C. W. Carter, Jr & R. M. Sweet, Eds., Academic Press).

Absorption correction: Sheldrick, G. M. SADABS - Bruker Nonius area detector scaling and absorption correction - V2.\0

Structure solution: SHELXS97 (G. M. Sheldrick, Acta Cryst. (1990) A46 467-473). Structure refinement: SHELXL97 (G. M. Sheldrick (1997), University of Göttingen, Germany)

Graphics: Cameron - A Molecular Graphics Package (D. M. Watkin, L. Pearce and C. K. Prout, Chemical Crystallography Laboratory, University of Oxford, 1993)

Special details: All hydrogen atoms were placed in idealised positions and refined using a riding model, except those of the NH and OH which were located in the difference map and refined using restraints. Chirality: NI=R, C12=S, C13=S, CI5=R, C21=S, C24=R

The results of the studies are set out below in Tables A, B, C, D and E.

In the Tables, the label RUS0350 refers to Isomer B.

**TABLE A**

| | | |
|---|---|---|
| Identification code | **2005bdy0585 (RUS03S0)** | |
| Empirical formula | C₂₉H₄₅NO₇S | 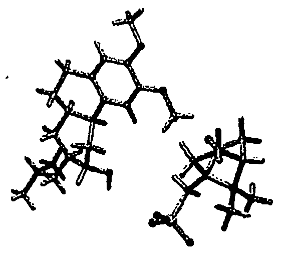 |
| Formula weight | 551.72 | |
| Temperature | 120(2) K | |
| Wavelength | 0.71073 Å | |
| Crystal system | Orthorhombic | |
| Space group | *P*2₁2₁2₁ | |
| Unit cell dimensions | *a* = 7.1732(9) Å | |
| | *b* =12.941(2) Å | |
| | *c* = 31.025(4) Å | |
| Volume | 2880.1(7) Å³ | |
| *Z* | 4 | |
| Density (calculated) | 1.272 Mg / m³ | |
| Absorption coefficient | 0.158 mm⁻¹ | |
| F(000) | 1192 | |
| crystal | Colourless Slab | |
| Crystal size | 0.2x02x0.04mm³ | |
| θ range for data collection | 3.06-27.37° | |
| Index ranges | -8 ≤ *h* ≤ 9, -16 ≤ *k* ≤ 16, -36 ≤ *l* ≤ 39 | |
| Reflections collected | 36802 | |
| Independent reflections | 6326 [*Rᵢₙₜ* = 0.0863] | |
| Completeness to θ =27.37° | 97.1 % | |
| Absorption correction | Semi-empirical from equivalents | |
| Max. and min. transmission | 0.9937 and 0.9690 | |
| Refinement method | Full-matrix least-squares on *F*² | |
| Data / restraints / parameters | 6326/1/357 | |
| Goodness-of fit on *F*² | 1.042 | |
| Final *R* indices [*F*² > 2σ(*F*²)] | *R1* = 0.0498, *wR2* = 0.0967 | |
| *R* indices (all data) | *R1* = 0.0901, *wR2* = 0,1108 | |
| Absolute structure parameter | 0.04(8) | |
| Extinction coefficient | 0.0059(7) | |
| Largest diff. peak and hole | 0.236 and -0.336 e Å⁻³ | |

**TABLE B. Atomic coordinates [x 10⁴], equivalent isotropic displacement parameters [A² x 10³] and site occupancy factors. U_{eq} is defined as one third of the trace of the orthogonalized U^{ij} tensor.**

| Atom | x | y | z | *Ueq* | *S.o.f*. |
|---|---|---|---|---|---|
| N1 | 4839(3) | 11119(2) | 2180(1) | 24(1) | 1 |
| 01 | 2515(3) | 13171(1) | 349(1) | 31(1) | 1 |
| 02 | 5581(3) | 14030(1) | 598(1) | 32(1) | 1 |
| 03 | 9220(3) | 12834(2) | 2385(1) | 36(1) | 1 |
| C1 | 870(4) | 12674(2) | 190(1) | 36(1) | 1 |
| C2 | 3176(3) | 12838(2) | 739(1) | 25(1) | 1 |
| C3 | 2346(4) | 12109(2) | 997(1) | 25(1) | 1 |
| C4 | 3124(3) | 11821(2) | 1395(1) | 24(1) | 1 |
| C5 | 4773(3) | 12276(2) | 1527(1) | 23(1) | 1 |
| C6 | 5629(4) | 13024(2) | 1262(1) | 24(1) | 1 |
| C7 | 4861(4) | 13308(2) | 875(1) | 25(1) | 1 |
| C8 | 7189(4) | 14582(2) | 747(1) | 38(1) | 1 |
| C9 | 2182(3) | 11023(2) | 1673(1) | 28(1) | 1 |
| C10 | 2759(3) | 11118(2) | 2137(1) | 26(1) | 1 |
| C11 | 5366(3) | 11096(2) | 2656(1) | 25(1) | 1 |
| C12 | 7292(4) | 11536(2) | 2747(1) | 25(1) | 1 |
| C13 | 7468(4) | 12663(2) | 2590(1) | 25(1) | 1 |
| C14 | 5988(4) | 12911(2) | 2252(1) | 25(1) | 1 |
| C15 | 5773(4) | 12010(2) | 1943(1) | 24(1) | 1 |
| C16 | 7734(4) | 11477(2) | 3232(1) | 28(1) | 1 |
| C17 | 7752(4) | 10418(2) | 3449(1) | 34(1) | 1 |
| C18 | 9198(6) | 9696(3) | 3249(1) | 65(1) | 1 |
| C19 | 8114(4) | 10562(2) | 3930(1) | 41(1) | 1 |
| C20 | 7509(4) | 8131 (2) | 1250(1) | 31(1) | 1 |
| S1 | 7409(1) | 8792(1) | 1754(1) | 27(1) | 1 |
| 04 | 7758(2) | 7965(1) | 2064(1) | 30(1) | 1 |
| 05 | 8831(3) | 9582(2) | 1760(1) | 49(1) | 1 |
| 06 | 5524(2) | 9221(1) | 1798(1) | 32(1) | 1 |
| 07 | 7406(3) | 6932(1) | 498(1) | 48(1) | 1 |
| C21 | 6858(3) | 8622(2) | 830(1) | 25(1) | 1 |
| C22 | 7154(4) | 7851(2) | 459(1) | 30(1) | 1 |
| C23 | 7073(4) | 8450(2) | 40(1) | 32(1) | 1 |
| C24 | 6648(3) | 9544(2) | 203(1) | 28(1) | 1 |
| C25 | 4742(3) | 8877(2) | 787(1) | 29(1) | 1 |
| C26 | 4742(3) | 8877(2) | 787(1) | 29(1) | 1 |
| C27 | 7773(4) | 9610(2) | 630(1) | 25(1) | 1 |
| C28 | 7431(4) | 10628(2) | 868(1) | 29(1) | 1 |
| C29 | 9895(4) | 9489(2) | 569(1) | 36(1) | 1 |

**TABLE C. Bond lengths [A] and angles [°].**

| | | | |
|---|---|---|---|
| N1-C10 | 1.498(3) | C14-C15 | 1.518(3) |
| N1-C15 | 1.522(3) | C16-C17 | 1.526(3) |
| N1-C11 | 1.524(3) | C17-C18 | 1.527(4) |
| 01-C2 | 1.368(3) | C17-C19 | 1.527(4) |
| O1-C1 | 1.432(3) | C20-C21 | 1,525(3) |
| 02-C7 | 1.369(3) | C20-S1 | 1.784(2) |
| 02-C8 | 1.433(3) | Sl-05 | 1,4442(19) |
| 03-C13 | 1.425(3) | Sl-04 | 1.4607(17) |
| C2-C3 | 1.372(3) | Sl-06 | 1.4676(18) |
| C2-C7 | 1.417(3) | 07-C22 | 1.208(3) |
| C3-C4 | 1.407(3) | C21-C22 | 1.537(4) |
| C4-C5 | 1.384(3) | C21-C26 | 1.559(3) |
| C4-C9 | 1.506(3) | C21-C27 | 1.565(3) |
| C5-C6 | 1.411(3) | C22-C23 | 1.517(4) |
| C5-C15 | 1.516(3) | C23-C24 | 1.535(4) |
| C6-C7 | 1.372(3) | C24-C25 | 1.548(4) |
| C9-C10 | 1.504(3) | C24-C27 | 1.554(4) |
| C11~C12 | 1.521(3) | C25-C26 | 1.557(4) |
| C12-C16 | 1.540(3) | C27-C28 | 1.529(3) |
| C12-C13 | 1.544(3) | C27-C29 | 1,542(4) |
| C13-C14 | 1.524(3) | | |
| | | | |
| C10-N1-C15 | 113.33(19) | C12-C11-N1 | 113.43(19) |
| C10-N1-C11 | 109.46(18) | C11-C12-C16 | 110.5(2) |
| C15-N1-C11 | 111.98(19) | C11-C12-C13 | 111.7(2) |
| C2-01-C1 | 116.6(2) | C16-C12-C13 | 109.84(19) |
| C7-02-C8 | 116.27(19) | 03-C13-C14 | 106.0(2) |
| 01-C2-C3 | 125.5(2) | 03-C13-C12 | 111.1(2) |
| 01-C2-C7 | 115.0(2) | C14-C13-C12 | 111.0(2) |
| C3-C2-C7 | 119.5(2) | C15-C14-C13 | 710.1(2) |
| C2-C3-C4 | 121.5(2) | C5-C15-C14 | 114.3(2) |
| C5-C4-C3 | 119.2(2) | C5-C15-N1 | 112.0(2) |
| C5-C4-C9 | 120.3(2) | C14-C15-N1 | 108.7(2) |
| C3-C4-C9 | 120.5(2) | C17-C16-C12 | 118.4(2) |
| C4-C5-C6 | 119.4(2) | C16-C17-C18 | 112.2(2) |
| C4-C5-C15 | 124.1(2) | C16-C-17-C19 | 108.7(2) |
| C6-C5-C15 | 118.6(2) | C8-C17-C19 | 110.8(3) |
| C7-C6-C5 | 121.3(2) | C21-C20-S1 | 122.51(18) |
| 02-C7-C6 | 125.4(2) | 05-S1-04 | 112.93(11) |
| 02-C7-C2 | 115.4(2) | 05-S1-06 | 112.47(12) |
| C6-C7-C2 | 119.2(2) | 04-S1-06 | 191.93(11) |
| C10-C9-C4 | 111.7(2) | 05-S1-C20 | 108.81(13) |
| N1-C10-C9 | 111.0(2) | 04-S1-C20 | 102.60(11) |
| | | | |
| 06-S1-C20 | 107.44(12) | C23-C24-C25 | 106.4(2) |
| C20-C21-C22 | 109.0(2) | C23-C24-C27 | 103.3(2) |
| C20-C21-C26 | 117.3(2) | C25-C24-C27 | 102.3(2) |
| C22-C21-C26 | 102.1(2) | C24-C25-C26 | 102.9(2) |
| C20-C21-C27 | 123.4(2) | C25-C26-C21 | 104.2(2) |
| C22-C29-C27 | 100.21(19) | C28-C27-C29 | 107.8(2) |
| C26-C21-C27 | 101.7(2) | C28-C27-C24 | 112.0(2) |
| 07-C22-C23 | 126.4(2) | C29-C27-C24 | 113,7(2) |
| 07-C22-C21 | 125.9(2) | C28-C27-C21 | 116.5(2) |
| C23-C22-C21 | 107.7(2) | C29-C27-C21 | 112.3(2) |
| C22-C23-C24 | 101.3(2) | C24-C27-C21 | 94.27(19) |

**TABLE D. Anisotropic displacement parameters [A²x 10³]_{.} The anisotropic displacement factor exponent takes the form:- 2π²[h²a*²U¹¹+ ... + 2 h k a* b* U¹²].**

| Atom | *U¹¹* | *U²²* | *U³³* | *U²³* | *U¹³* | *U¹²* |
|---|---|---|---|---|---|---|
| N1 | 26(1) | 24(1) | 23(1) | 2(1) | -1(1) | -3(1) |
| 01 | 37(1) | 30(1) | 24(1) | 3(1) | -7(1) | -4(1) |
| 02 | 41(1) | 31(1) | 25(1) | 5(1) | -2(1) | -10(1) |
| 03 | 26(1) | 49(1) | 32(1) | 7(1) | -3(1) | -9(1) |
| C1 | 41(2) | 36(2) | 32(2) | 3(1) | -9(1) | -8(2) |
| C2 | 30(2) | 24(2) | 22(1) | 1(1) | -1(1) | 2(1) |
| C3 | 25(1) | 26(1) | 24(1) | -3(1) | -2(1) | 2(1) |
| C4 | 26(2) | 22(1) | 23(1) | -1(1) | 2(1) | -1(1) |
| C5 | 24(1) | 22(1) | 23(1) | -2(1) | 1(1) | 0(1) |
| C6 | 26(1) | 22(1) | 24(1) | -3(1) | 2(1) | -5(1) |
| C7 | 30(2) | 22(1) | 22(1) | 2(1) | 4(1) | -4(1) |
| C8 | 45(2) | 34(2) | 36(2) | 5(1) | -2(1) | -20(2) |
| C9 | 23(1) | 32(1) | 29(2) | 3(1) | -1(1) | -4(1) |
| C10 | 26(1) | 29(1) | 25(1) | 2(1) | 0(1) | -5(1) |
| C11 | 31(1) | 25(1) | 20(1) | 2(1) | 0(1) | -2(1) |
| C12 | 26(1) | 26(1) | 23(1) | -1(1) | 1(1) | -1(1) |
| C13 | 26(1) | 28(1) | 23(1) | -1(1) | -1(1) | -2(1) |
| C14 | 30(2) | 22(2) | 24(1) | -1(1) | 1(1) | -1(1) |
| C15 | 22(1) | 22(1) | 28(1) | 2(1) | 0(1) | -4(1) |
| C16 | 31(1) | 28(1) | 24(1) | -1(1) | -3(1) | 3(1) |
| C17 | 46(2) | 31(2) | 25(1) | 1(1) | -7(1) | 0(2) |
| C18 | 106(3) | 46(2) | 41(2) | 6(2) | -1(2) | 31(2) |
| C19 | 51(2) | 41(2) | 31(2) | 9(2) | -7(1) | -4(2) |
| C20 | 30(2) | 34(2) | 29(1) | 2(1) | 3(1) | 9(2) |
| S1 | 27(1) | 30(1) | 24(1) | 4(1) | -2(1) | -5(1) |
| O4 | 31(1) | 36(1) | 23(1) | 9(1) | -1(1) | 0(1) |
| O5 | 53(1) | 58(1). | 37(1) | 13(1) | -11(1) | -35(1) |
| O6 | 34(1) | 35(1) | 28(1) | -3(1) | -2(1) | 10(1) |
| O7 | 81(2) | 25(1) | 40(1) | -1(1) | 12(1) | 6(1) |
| C21 | 25(1) | 25(2) | 24(1) | -1(1) | 3(1) | 2(1) |
| C22 | 35(2) | 25(2) | 31(2) | 0(1) | 1(1) | -1(1) |
| C23 | 40(2) | 30(2) | 25(1) | -2(1) | 1(1) | -2(1) |
| C24 | 28(1) | 29(2) | 26(2) | 2(1) | 2(1) | 2(1) |
| C25 | 30(2) | 34(2) | 29(2) | -1(1) | -2(1) | 0(1) |
| C26 | 26(1) | 34(2) | 28(2) | 0(1) | 1(1) | -5(1) |
| C27 | 23(1) | 26(1) | 26(1) | 0(1) | 2(1) | 0(1) |
| C28 | 31(1) | 26(1) | 30(1) | 0(1) | -2(1) | -6(1) |
| C29 | 29(2) | 41(2) | 40(2) | 0(2) | 2(1) | -3(1) |

**TABLE E. Hydrogen coordinates [x 10⁴] and isotropic displacement parameters [A² x 10³].**

| Atom | *x* | *y* | *z* | *U_{eq}* | *S.o.f* |
|---|---|---|---|---|---|
| | | | | | |
| H98 | 5190(40) | 10528(15) | 2062(10) | 70(8) | 1 |
| H99 | 10030(50) | 12950(30) | 2575(12) | 70(8) | 1 |
| H1A | 1107 | 11933 | 156 | 54 | 1 |
| H1B | 529 | 12973 | -89 | 54 | 1 |
| H1C | -154 | 12777 | 395 | 54 | 1 |
| H3 | 1220 | 11793 | 904 | 30 | 1 |
| H6 | 6760 | 13337 | 1353 | 29 | 1 |
| H8A | 6872 | 14966 | 1009 | 58 | 1 |
| H8B | 7600 | 95065 | 523 | 58 | 1 |
| H8C | 8193 | 14091 | 810 | 58 | 1 |
| H9A | 814 | 11108 | 1651 | 33 | 1 |
| H9B | 2505 | 10324 | 1567 | 33 | 1 |
| H10A | 2250 | 11767 | 2259 | 32 | 1 |
| H10B | 2235 | 10534 | 2304 | 32 | 1 |
| H11A | 4431 | 11494 | 2822 | 30 | 1 |
| H11B | 5322 | 70372 | 2759 | 30 | 1 |
| H12 | 8230 | 11108 | 2589 | 30 | 1 |
| H13 | 7334 | 13145 | 2840 | 30 | 1 |
| H14A | 4783 | 13050 | 2397 | 30 | 1 |
| H14B | 6354 | 13538 | 2090 | 30 | 1 |
| H15 | 7056 | 11776 | 1864 | 29 | 1 |
| H16A | 8973 | 11796 | 3278 | 33 | 1 |
| H16B | 6813 | 11911 | 3386 | 33 | 1 |
| IH17 | 6493 | 10088 | 3412 | 41 | 1 |
| H18A | 8906 | 9588 | 2944 | 97 | 1 |
| H18B | 9176 | 9031 | 3400 | 97 | 1 |
| H18C | 10440 | 10005 | 3276 | 97 | 1 |
| H19A | 9329 | 10894 | 3971 | 62 | 1 |
| H19B | 8110 | 9887 | 4073 | 62 | 1 |
| H19C | 7135 | 10999 | 4054 | 62 | 1 |
| H20A | 8824 | 7924 | 1207 | 37 | 1 |
| H20B | 6787 | 7484 | 1286 | 37 | 1 |
| H23A | 6070 | 8190 | -151 | 38 | 1 |
| H23B | 8277 | 8423 | -116 | 38 | 1 |
| H24 | 6928 | 10107 | -8 | 33 | 1 |
| H25A | 3773 | 9195 | 153 | 37 | 1 |
| H25B | 4152 | 10235 | 426 | 37 | 1 |
| H26A | 3994 | 8237 | 764 | 35 | 1 |
| H26B | 4300 | 9279 | 1039 | 35 | 1 |
| H28A | 8160 | 10638 | 1135 | 44 | 1 |
| IH28B | 6103 | 10692 | 936 | 44 | 1 |
| H28C | 7811 | 11207 | 684 | 44 | 1 |
| H29A | 10358 | 10042 | 381 | 54 | 1 |
| H29B | 10159 | 8817 | 436 | 54 | 1 |
| H29C | 10517 | 9531 | 849 | 54 | 1 |

**Table 6. Hydrogen bonds [Å and °].**

| *D*-H..*A* | *d*(*D*,H) | *d*(H···*A*) | *d*(*D···A*) | ∠(*D*H*A*) |
|---|---|---|---|---|
| | | | | |
| N1-H98···O6 | 0.885(10) | 1.895(12) | 2.773(3) | 171(3) |
| N1-H98···S1 | 0.885(10) | 2.914(14) | 3.771(2) | 163(3) |
| 03-H99···O4ⁱ | 0.8A(4) | 1.94(4) | 2.766(3) | 165(3) |
| O3-H99···S1ⁱ | 0.84(4) | 2.98(4) | 3.811(2) | 169(3) |
| Symmetry transformations used to generate equivalent atoms: | | | | |
| (i)-x+2,y+1/2, -z+1/2 | | | | |

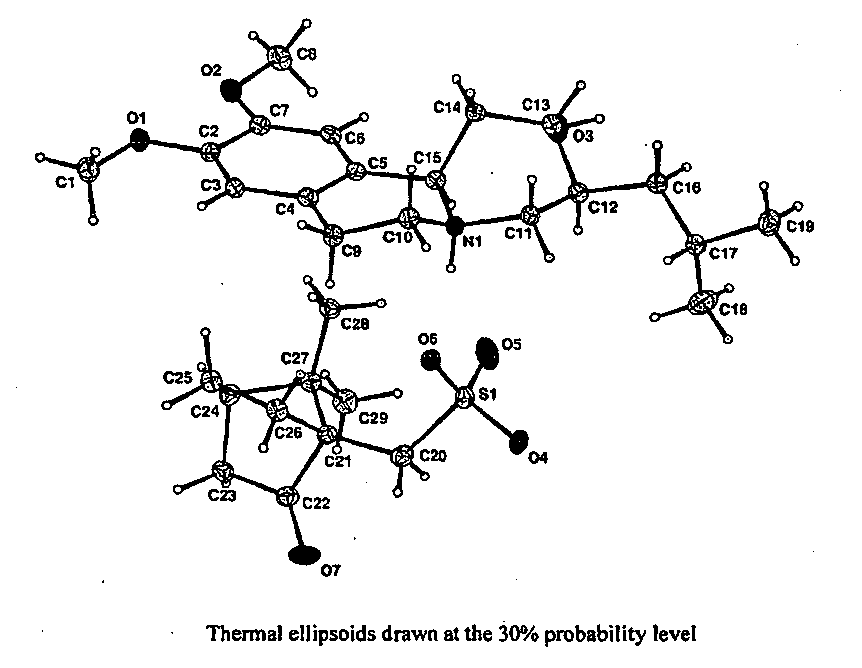

On the basis of the data set out above, Isomer B is believed to have the 2S, 3S, 11bR configuration, which corresponds to Formula (Ia):

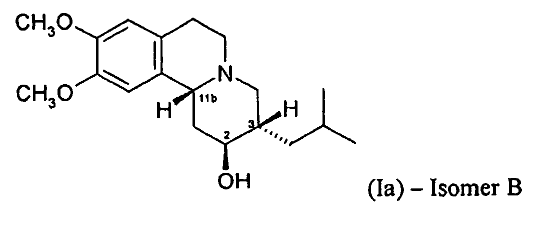

### EXAMPLE 5

### Analysis of the effect of Isomer B in a transgenic mice model of Huntington's disease

B6CBA-Tg(HDexon1)62Gpb/1J transgenic (R6/2) mice are transgenic for the 5-end of the human HD gene carrying (CAG) 115- (CAG) 150 repeat expansions. R6/2 transgenic mice exhibit a progressive neurological phenotype that mimics many of the features of Huntington's disease, including choreiforin-like movements, involuntary stereotypic movements, tremor and epileptic seizures. They urinate frequently and exhibit loss of body weight through the course of the disease. These symptoms appear between 6 and 8 weeks of age.

A study was carried out to assess the effect, of Isomer B in this transgenic mouse model of Huntington's disease by assessing animals in a battery of behavioural tests.

### Methods

Female B6CBa-Tg(HDexon1)62Gpb/1J transgenic mice (Jackson Laboratory, USA) were housed 5 per cage in an enriched environment under a light-dark cycle of 12 h - 12 h (light on at 7.00 am, off at 7.00 pm) at a room temperature of 21 ± 2 °C, with 50±15% humidity. The mice had access to commercial mouse chow (mouse/rate bleeding, ref. 9341 Provimi Kliba, Switzerland) and tap water.

Isomer B in corn oil was administered repeatedly (5 mg/kg i.p) once per day for 4 days to 10 weeks old mice.

On the testing days, animals were subjected to the following tests, using the protocol described in the protocol section:

### 1. Simplified Irwin test

Two hours before the observations, animals were placed in individual cages. Measurements were first carried out in the individual cages. The animal's convulsions, tremors-twitches, stereotypies and vocalizations were recorded. The animal was then placed in a 58.5 x 68.5 cm open field with a 6 cm rim and observed for approximately 3 minutes. Gait characteristics were ranked. The presence of convulsions or tremors-twitches and stereotypies was again noted. At the end of the 3 minutes, the number of faecal boluses and pools of urine was recorded. The mice were then returned to their individual cages after testing.

### 2. Locomotor activity

The mice were placed in a transparent plastic box of floor dimensions 30 x 30 cm in a room with low light intensity (maximum 20 lux). Locomotor activity was determined during a 10 minute period using a video image analyzer (Videotrack, View Point, Lyon, France). The number, distance and average speed of ambulatory movements were measured. The mice were returned to their home cages after testing.

### 3. Rotarod

Two consecutive days before the first administration, animals were trained to use the rotarod: they were placed on an accelerating rotarod (Ugo Basile, Italy) for a maximum time of 450 seconds. They went through 2 training sessions starting with 4 rpm for 300 seconds, then staying at 40 rpm for 150 seconds. The two training sessions were given at 1 hour intervals. On the days of testing, each animal was subjected to one trial under the conditions described above. Each trial was terminated when the mouse fell or when it had stayed on the rotarod for 450 seconds. The mice were returned to their home cages after testing.

The results of the tests are shown in Tables 5 to 9.

### Experimental protocol

### Size of experimental groups: 10

Group 1: hemizygote Transgenic B6CBA-Tg(HDexon 1)62Gpb/J mice treated with vehicle i.p. once a day for 4 days (from day 0 to day 3)
Group 2: hemizygote Transgenic B6CBA-Tg(HDexon i)62Gpb/1J mice treated with 5 mg/kg i.p. of test item once a day for 4 days (from day 0 to day 3)

### Testing protocol

At the age of 10 weeks, before administration (Day 0) and each day for 3 days following administration, animals were subject to the tests described below, as follows:

### Day-2

- Rotarod training, 2 sessions at 1 hour interval

### Day -1

- Rotarod training, 2 sessions at 1 hour interval

### Day 0

- Simplified Irwin test
- Locomotor activity test, immediately after the simplified Irwin test
- Rotarod test, immediately after the locomotor activity test
- Test item administration, 1 hour after the rotarod test
- Simplified Irwin test, 40 minutes after administration
- Locomotor activity, immediately after the simplified Irwin test

### Day 1

- Test item administration
- Simplified Irwin test, 40 minutes after administration
- Rotarod test, immediately after the simplified Irwin test

### Day 2

- Test item adminstation
- Simplified Irwin test, 40 min after administration
- Rotarod test, immediately after simplified #Irwin test

### Day 3

- Test item administration
- Simplified Irwin test, 40 min after administration
- Locamotor activity test, immediately after the simplified Irwin test

### Statistical analysis

Simplified Irwin scores were analysed using non-parametric Mann-Whitney's U test. Locomotor activity data were analysed using Dunnett's t-test. Rotarod scores were analysed using the non-parametric Mann-Whitney's U-test. Statistical analyses were performed using the software Statview SE^{+graphics} software, Brain Power.

**Table 5- Effects of Isomer B in a transgenic mice model of Huntington's disease Phase I : Efficacy in acute study, administration from Day 0 to Day 3 - Simplified Irwin test**

| **Corn oil, 10 ml/kg i.p.** | Day 0 before administration | Day 0 after administration | Day 1 | Day 2 | Day 3 | Day 17 | Day 21 | Day 24 | Day 27 | Day 31 |
|---|---|---|---|---|---|---|---|---|---|---|
| **HOME CAGE** | | | | | | | | | | |
| Convulsions | - | - | - | 0.78 ± 0.40 | 0.67 ± 0.44 | X | X | X | X | X |
| Tremors-witches | 1.33 ± 0.17 | 1.00 ± 0.27 | 0.78 ± 0.22 | 0.88 ± 0.13 | 1.44 ± 0.29 | X | X | X | X | X |
| Stereotypies | - | - | - | 0.11 ± 0.11 | - | X | X | X | X | X |
| Grooming (sec) | 21.00 ± 8.49 | 4.00 ± 4.00 | 1.33 ± 1.33 | 14.22 ± 8.18 | 11.67 ± 7.72 | X | X | X | X | X |
| Vocalization | - | - | - | - | - | X | X | X | X | X |
| | | | | | | | | | | |
| **OPEN FIELD** | | | | | | | | n=2/8 | n=2/8 | n=3/7 |
| Convulsions | - | - | - | - | - | - | - | - | - | - |
| Tremors-Twitches | 2.11 ± 0.26 | 1.89 ± 0.11 | 2.22 ± 0.15 | 1.89 ± 0.20 | 1.67 ± 0.24 | 2.78 ± 0.15 | 2.69 ± 0.29 | 3.38 ± 0.25 | 2.00 ± 0.25 | 2.71 ± 0.25 |
| Stereotypies | - | - | - | - | - | - | - | - | - | - |
| Grooming (sec) | 18.89 ± 10.50 | 40.89 ± 15.82 | 26.00 ± 13.43 | 45.67 ± 16.53 | 44.33 ± 9.07 | 24.67 ± 8.18 | 20.63 ± 8.90 | 29.50 ± 8.67 | 30.63 ± 12.5 | 21.29 ± 9.53 |
| Gait | 1.22 ± 0.46 | 0.89 ± 0.35 | 0.56 ± 029 | 1.33 ± 0.47 | 2.33 ± 0.24 | 2.89 ± 0.11 | 2.73 ± 0.15 | 2.88 ± 0.28 | 2.25 ± 0.50 | 3.14 ± 0.30 |
| VocalisationVocalisation | - | - | - | - | - | 0.22 ± 0.22 | 0.50 ± 0.31 | 0.25 ± 0.24 | 0.50 ± 0.31 | 1.14 ± 0.36 |
| Defecation (No of boluses) | 1.67 ± 0.50 | 1.33 ± 0.29 | 1.00 ± 0.33 | 1.67 ± 0.29 | 2.22 ± 0.40 | 1.11 0.56 | 1.75 ± 0.43 | 1.25 ± 0.39 | 1.50 ± 0.31 | 1.57 ± 0.42 |
| Urination (N° of pools) | 0.44 ± 0.24 | 0.22 ± 0.15 | 0.33 ± 0.24 | 0.22 ± 0.15 | 0.11 ± 0.11 | 0.22 ± 0.15 | 0.50 ± 0.18 | 0.13 0.12 | 0.00 ± 0.00 | 0.00 ± 0.00 |
| | | | | | | | | | | |

| **Isomer B 5 mg/kg i.p.** | Day 0 before administration | Day 0 after administration | Day 1 | Day 2 | Day 3 | Day 17 | Day 21 | Day 24 | Day 27 | Day 31 |
|---|---|---|---|---|---|---|---|---|---|---|
| **HOME CAGE** | | | | | | | | | | |
| Convulsions | 0.22 ± 0.22 | 0.13 ± 0.13 | - | - | 0.11 ± 0.11 | X | X | X | X | X |
| Tremors-Twitches | 1.44 ± 0.24 | 1.13 ± 0.30 | 1.00 ± 0.24 | 0.78 ± 0.15 | 1.00 ± 0.29 | X | X | X | X | X |
| Stereotypies | - | - | - | - | - | X | X | X | X | X |
| Grooming (sec) | 10.00 ± 7.07 | 7.38 ± 6.54 | - | 7.78 ± 6.13 | 4.22 ± 4.22 | X | X | X | X | X |
| Vocalization | - | - | - | - | - | X | X | X | X | X |
| | | | | | | | | | | |
| **OPEN FIELD** | | | | | | | | | | |
| Convulsions | - | 0.67 ± 0.67 | 0.11 ± 0.11 | - | - | 0.67 ± 0.67 | - | - | | - |
| Tremors-Twitches | 2.11 ± 011 | 1.89 ± 0.26 | 2.33 ± 0.17 | 2.00 ± 0.24 | 1.78 ± 0.36 | 1.44 ± 0.24* | 1.67 ± 0.22* | 2.44 ± 0.18* | 2.11 ± 0.39 | 2.67 ± 0.24 |
| Stereotypies | - | 0.11±0.11 | - | - | - | - | - | - | - | - |
| Grooming (sec) | 19.44 ± 6.92 | 3.11 ± 2.45 | 7.78 ± 4.34 | 20.56 ± 6.94 | 12.67 ± 6.71** | 20.33 ± 7.49 | 17.67 ± 6.33 | 18.22 ± 8.25 | 25.33 ± 8.14 | 19.11 ± 8.71 |
| Gait | 1.11 ± 0.39 | 0.33 ± 0.24 | 1.00 ± 0.33 | 0.67 ± 0.37 | 2.33 ± 0.33 | 1.56 ± 0.34* | 1.67 ± 0.37* | 2.17 ± 0.12^{a} | 2.56 ± 0.24 | 2.33 ± 0.29 |
| Vocalisation | 0.44 ± 029 | 0.22 ± 0.22 | 0.67 ± 0.33* | 0.89 ± 0.35 | 0.89 ± 0.35* | 0.22 ± 0.22 | 0.44 ± 0.29 | 0.44 ± 0.29 | 0.00 ± 0.00 | 0.22 ± 0.22 |
| Defecation (N° of boluses) | 2.56 ± 0.50 | 1.44 ± 0.50 | 0.89 ± 0.26 | 1.22 ± 0.43 | 1.33 ± 0.33 | 2.67 ± 0.76 | 2.44 ± 0.53 | 1.33 ± 0.55 | 2.33 ± 0.58 | 1.44 ± 0.38 |
| Urination (N° of pools) | 0.67 ± 0.44 | 0.44 ± 0.34 | 0.11 ± 0.11 | 0.33 ± 0.24 | 0.11 ± 0.11 | 0.22 ± 0.15 | 0.11 ± 0.11 | 0.33 ± 0.17 | 0.44 ± 0.18* | 0.11± 0.11 |

| | | | | | | | | | | |
|---|---|---|---|---|---|---|---|---|---|---|
| —: Sign never observed (equivalent to 0.00 ± 0.00) a: p = 0.06 versus control group, Mann-Whitney U-test X : Observation not performed * ; ** : Significantly different from control group (p<0.05; p<0.01), Mann-Whitney U-test | | | | | | | | | | |

**Table 6**

| **Effects of Isomer B in a transgenic mice model of Huntington's disease** **Phase I : Efficacy in acute study - Locomotor activity test** **Average recorded results** | | | | |
|---|---|---|---|---|
| **DAY 0, before administration** | | | | |
| | | | | |
| | **Inactivity** | **Large movements** | | |
| Treatment | *Duration* | *Occurence* | *Duration* | *Speed* |
| | *(sec)* | *(number)* | *(sec)* | *(cm*/*sec)* |
| Corn oil, 10 mL/kg i.p. | 140 ± 17 | 292 ± 34 | 126 ± 17 | 14.2 ± 0.8 |
| RUS-350, 5 mg/kg i.p. | 149 ± 27 | 309 ± 37 | 128 ± 16 | 13.2 ± 0.5 |

| | | | | |
|---|---|---|---|---|
| **DAY 0,40 mn after administration** | | | | |
| | | | | |
| | **Inactivity** | **Large movements** | | |
| Treatment | *Duration* | *Occurence* | *Duration* | *Speed* |
| | *(sec)* | *(number)* | *(sec)* | *(cm*/*sec)* |
| Corn oil, 10 mL/kg i.p. | 199 ± 16 | 205 ± 14 | 84 ± 8 | 14.0 ± 0.7 |
| RUS-350, 5 mg/kg i.p. | 184 ± 31 | 280 ± 41 | 99 ±13 | 13.0 ± 0.5 |

| | | | | |
|---|---|---|---|---|
| **DAY 2,40 mn after administration** | | | | |
| | | | | |
| | **Inactivity** | **Large movements** | | |
| Treatment | *Duration* | *Occurence* | *Duration* | *Speed* |
| | *(sec)* | *(number)* | *(see)* | *(cm*/*sec)* |
| Corn oil, 10 mL/kg i.p. | 171 ± 15 | 245 ± 22 | 104 ± 9 | 14.1 ± 0.7 |
| RUS-350, 5 mg/kg i.p. | 134 ± 18 | 322 ± 40 | 123 ± 14 | 13.9 ± 0.7 |

**Table 7**

| **Effects of Isomer B in a transgenic mice model of Huntington's desease** **Phase I : Efficacy in acute study - Locomotor activity test** **Average results relative to pre-administration** | | | | |
|---|---|---|---|---|
| **DAY 0, before administration** | | | | |
| | | | | |
| | **Inactivity** | **Large movements** | | |
| Treatment | *Duration* | *Occurrence* | *Duration* | *Speed* |
| | *(see)* | *(number)* | *(sec)* | *(cm*/*sec)* |
| Corn oil, 10 mL/kg i.p. | 0 ± 0 | 0 ± 0 | 0 ± 0 | 0.0 ± 0.0 |
| RUS-350, 5 mg/kg i.p. | 0 ± 0 | 0 ± 0 | 0 ± 0 | 0.0 ± 0.0 |

| | | | | |
|---|---|---|---|---|
| **DAY 0, 40 mn after administration** | | | | |
| | | | | |
| | **inactivity** | **Large movements** | | |
| Treatment | *Duration* | *Occurence* | *Duration* | *Speed* |
| | *(sec)* | *(number)* | *(sec)* | *(cm*/*sec)* |
| Corn oil, 10 mL/kg i.p. | 60 ± 20 | -87 ± 24 | -42 ± 10 | -0.2 ± 0.4 |
| RUS-350, 5 mg/kg i.p. | 36 ± 30 | -29 ± 41 | -29 ± 15 | -0.1 ± 0.5 |

| | | | | |
|---|---|---|---|---|
| **DAY 2, 40 mn after administration** | | | | |
| | | | | |
| | **Inactivity** | **Large movements** | | |
| Treatment | *Duration* | *Occurrence* | *Duration* | *Speed* |
| | *(sec)* | *(number)* | *(sec)* | *(cm*/*sec)* |
| Corn oil, 10 mL/kg i.p. | 31 ± 10 | -47 ± 23 | -21 ± 11 | -0.1 ± 0.7 |
| RUS-350, 5 mg/kg i.p. | -15 ± 27 | 13 ± 42 | -6 ± 16 | 0.7 ± 0.7 |

**Table 8**

| **Effects of Isomer B in a transgenic mice model of Huntington's desease** **Phase I : Efficacy in acute study - Locomotor activity test** **Average recorded results** | | | | |
|---|---|---|---|---|
| **DAY 24** | | | | |
| | | | | |
| | **Inactivity** | **Large movements** | | |
| Treatment | *Duration* | *Occurence* | *Duration* | *Speed* |
| | *(sec)* | *(number)* | *(sec)* | *(cm*/*sec)* |
| Corn oil, 10 mL/kg i.p. | 36 ± 20 | 741 ± 152 | 136 ± 22 | 11.8 ± 0.6 |
| RUS-350, 5 mg/kg i.p. | 67 ± 42 | 818 ± 168 | 148 ± 34 | 11.2 ± 0.9 |

**Table 9**

| **Effects of Isomer B in a transgenic mice model of Huntington's desease** **Phase I : Efficacy in acute study, administration from Day 0 to Day 3 -** **Rotarod test** | | | | | | |
|---|---|---|---|---|---|---|
| Measured latencies to fall from Rotarod (sec) after daily administration of Isomer B | | | | Latencies relative to Day 0 (prior to Ist administration) | | |
| | Corn oil,10 mL/kg i.p. | Isomer B,5 mg/kg i.p. | | | Corn oil, 10 mL/kg i.p. | Isomer B,5 mg/kg i.p. |
| Day 0, prior to 1st administration | 76.9 ± 16.6 | 68.4 ± 17.7 | | Day 1 - Day 0 | -0.7 ± 13.0 | 23.0 ± 9.8 |
| Day 1 | 76.2 ± 18.3 | 91.4 ± 18.1 | | Day 3 - Day 0 | -24.0 ± 10.9 | -9.0 ± 11.9 |
| Day 3 | 52.9 ± 15.7 | 59.4 ± 11.2 | | Day 9 - Day 0 | -32.1 ± 9.7 | -39.7 ± 9.9 |
| Day 9 | 46.0 ± 13.1 | 29.0 ± 1.2.1 | | Day 17 - Day 0 | -51.3 ± 15.2 | -38.2 ± 13.1 |
| Day 17 | 26.0 ± 9.2 | 30.0 ± 11.0 | | Day 21 - Day 0 | -64.3 ± 13.3 | -46.3 ± 15.5 |
| Day 21 | 18.5 ± 8.6 | 22.1 ± 7.3 | | Day 24 - Day 0 | -61.9 ± 12.0 | -53.7 ± 16.8 |
| Day 24 | 20.9 ± 11.1 | 14.8 ± 4.3 | | Day 27- Day 0 | -62.6 ± 13.1 | -52.7 ± 16.2 |
| Day 27 | 20.1 ± 11.9 | 15.8 ± 5.2 | | Day 31 - Day 0 | -72.3 ± 16.4 | -60.7 ± 16.2 |
| Day 31 | 16.1 ± 9.8 | 7.8 ± 2.4 | | | | |

The results demonstrate that although both control mice and mice treated with Isomer B both exhibited some progression in the symptoms typical of Huntington's disease during the first three days following administration, the mice treated with Isomer B exhibited significantly less deterioration than the control mice during the period of 17 to 24 days after administration. In particular, deterioration in gait was substantially arrested or slowed during this period, and the incidences of involuntary movements such as involuntary chorea, tremors and twitches in the Isomer B-treated mice were no worse after 21 days than they had been prior to administration of the Isomer B. It is conceivable that by repeating the administration of Isomer B at appropriate intervals (which was not done in the tests), the development of the symptoms could be arrested or slowed still further.

Thus, the results indicate that Isomer B would be useful in preventing the onset of, or slowing the development of, the symptoms associated with Huntington's disease.

### EXAMPLE 6

### Comparison of the Sedative Properties of Tetrabenazine and the Dihydrotetrabenazine Isomers B and C

A study was carried out in rats to determine whether the dihydrotetrabenazine isomers of the invention have sedative properties. The effects of the isomers on spontaneous locomotor activity in rats were compared with the effects produced by tetrabenazine and haloperidol using the methods set out below. The results are shown in Table 10.

### Methods

Male Sprague-Dawley rats, (Charles River Laboratories, Saint-Germain/L'Arbresle, France), weighing 200-250 g at the beginning of the study, were used for the studies. The rats were housed, 2 or 3 per cage, in Makrolon type III cages, in a room set up with the following environmental conditions: temperature; 20 ± 2 °C, humidity : minimum 45 %, air changes: > 12 per hour, light/dark cycle of 12 h/12 h [on at 7:00 a.m.]. The rats were allowed to acclimatize to their conditions for at least five days before commencement of the study. The rats received food (Dietex, Vigny, France, ref. 811002) and water (tap water in water bottle) *ad libitum*.

Solutions of each test compound in corn oil were freshly prepared on the day of the experiment. Haloperidol was prepared in hydroxyethylcellulose, 0.5% in deionized water. Either the vehicle or the test compounds were administered as a single dose (0.3, 1,3 and 10 mg/kg, 2 mL/kg i.p.). The reference compound haloperidol (1 mg/kg) was administered i.p. (2 mL/kg).

The animals were placed in plexiglass cages under a video camera in a room with low light intensity (maximum 50 lux). At forty five minutes and 3 hours after administration, locomotor activity was determined during 20 minute periods using a video image analyzer (Videotrack, View Point, France). Locomotor activity was recorded in the reference group (haloperidol) at 1 hour after administration. The number and duration of ambulatory movements and duration of inactivity was measured. At the end of the locomotor activity measurement (45 minutes and 3 hours), palpebral closure and arousal were be scored as follows in the plexiglass cage:
Palpebral closure:
   - 0:: (normal) eyelids wide open
   - 1 :: eyelids slightly drooping
   - 2 :: ptosis, drooping eyelids approximately half-way
   - 3 :: eyelids completely shut
Arousal:
   - 1 :: very low, stupor, coma, little or no responsiveness
   - 2 :: low, some stupor, « dulled », some head or body movement
   - 3 :: somewhat low, slight stupor, some exploratory movements with periods of immobility
   - 4 :: normal, alert, exploratory movements /slow freeze
   - 5 :: somewhat high, slight excitement, tense, sudden darting or freezing
   - 6 :: very high, hyper alert, excited, sudden bouts of running or body movements

The number of occurrences and duration (in seconds) of ambulatory (large) movements and the duration of periods of inactivity (seconds) was determined during two 20 minute periods (45 minutes and 3 hours after administration) using a video image analyzer (Videotrack, ViewPoint, Lyon, France). Image tracking was performed using a video camera placed above the plexiglass cage, recording overall locomotor activity. Images recorded with the video camera were digitalized and displacement of the centre of gravity of the digital image spots was tracked and analyzed using the following method: the speed of displacement of the centre of gravity of the spot was measured and two threshold values were set to define the type of movement: threshold 1 (high speed) and threshold 2 (low speed). When the animal moved and the speed of displacement of the centre of gravity of the spot was above threshold 1, the movement was considered as an ambulatory movement. When the animal remained inactive, the speed was below threshold 2, the movement was considered as inactivity.

The results were expressed as the means SEMs of the 12 individual values. Statistical analyses were carried out using ANOVA (one way) and Dunnett's t-test and with the non parametric test of Kruskal-Wallis followed by a Mann & Whitey U-test for the sedation cotation. A p value of p<0,05 was taken as indicating significance.

### Protocol

### Group size n=12

- Group 1:: References haloperidol (1 mg/kg i.p.)
- Group 2:: Vehicle control group (2 ml/kg i.p.)
- Group 3:: tetrabenazine (0.3 mg/kg i.p)
- Group 4:: tetrabenazine (1 mg/kg i.p)
- Group 5:: tetrabenazine (3 mg/kg i.p)
- Group 6:: tetrabenazine (10 mg/kg i.p)
- Group 7:: Isomer C (0.3 mg/kg i.p)
- Group 8:: Isomer C (1 mg/kg i.p)
- Group 9:: Isomer C (3 mg/kg i.p)
- Group 10:: Isomer C (10 mg/kg i.p)
- Group 11:: Isomer B (0.3 mg/kg i.p)
- Group 12:: Isomer B (1 mg/kg i.p)
- Group 13:: Isomer B (3 mg/kg i.p)
- Group 14:: Isomer B (10 mg/kg i.p)

### Results

| Table 10 | | | | |
|---|---|---|---|---|
| Effects of Tetrabenazine, Isomer B, Isomer C (0.3,1,3 and 10 mg/kg i.p.) on spontaneous locomotor activity in rats | | | | |
| Observation time : 45 minutes after administration | | | | |
| | | **Large movements** | | **Inactivity** |
| **Treatment** | **Dose (mg/kg)** | **Occurrence** | **Duration (sec)** | **Duration (sec)** |
| Vehicle | 2mL/kg | 286 ± 35 | 76.4 ± 10.9 | 349.0 ± 37.4 |
| Haloperidol | 1 mg/kg | 58 ± 33 ** | 14.8 ± 8.5 ** | 637.2 ± 60.1 ** |
| Tebrabenazine | 0.3 mg/kg | 253 ± 32 | 66.8 ± 10.7 | 390.4 ± 37.4 |
| Tetrabenazine | 1 mg/kg | 189 ± 32 | 46.5 ± 8.6 | 456.5 ± 50.5 |
| Tetrabenazine | 3 mg/kg | 38 ± 25 ** | 8.7 ± 5.9 ** | 697.8 ± 39.7 ** |
| Tetrabenazine | 10 mg/kg | 1 ± 1 ** | 0.2 ± 0.2 ** | 723.1 ± 46.5 ** |
| Isomer C | 0.3 mg/kg | 285 ±34 | 79.2 ± 10.0 | 323.7 ± 25.6 |
| Isomer C | 1 mg/kg | 295 ± 30 | 71.8 ± 8.3 | 324.6 ± 38.1 |
| Isomer C | 3 mg/kg | 308 ± 36 | 84.0 ± 9.4 | 322.7 ± 27.8 |
| Isomer C | 10 mg/kg | 254 ± 32 | 66.5 ± 9.9 | 368.7 ± 30.9 |
| Isomer B | 0.3 mg/kg | 268 ± 36 | 72.0 ± 9.6 | 346.1 ± 36.9 |
| Isomer B | 1 mg/kg | 297 ± 22 | 87.0 ± 7.6 | 334.0 ± 23.2 |
| Isomer B | 3 mg/kg | 313 ± 38 | 89.1 ± 12.4 | 342.2 ± 33.3 |
| Isomer B | 10 mg/kg | 298 ± 37 | 84.0 ± 11.2 | 333.1 ± 26.9 |

| Observation time : 3 hours after administration | | | | |
|---|---|---|---|---|
| | | **Large movements** | | **Inactivity** |
| Treatment | Dose (mg/kg) | Occurrence | Duration (sec) | Duration (sec) |
| Vehicle | 2mL/kg | 101 ± 23 | 24.8 ± 6.0 | 540.9 ± 37.5 |
| Haloperidol | 1 mg/kg | 9 ± 8 ** | 2.2 ± 2.0 ** | 723.6 ± 50.2 ** |
| Tetrabenazine | 0.3 mg/kg | 96 ± 14 | 24.3 ± 4.2 | 545.9 ± 37.1 |
| Tetrabenazine | 1 mg/kg | 90 ± 16 | 21.5 ± 4.0 | 556.9 ± 31.1 |
| Tetrabenazine | 3 mg/kg | 9 ± 4 ** | 1.7 ± 0.9** | 729.9 ± 26.8 ** |
| Tetrabenazine | 10 mg/kg | 3 ± 1 ** | 0.6 ± 0.3 ** | 762.1 ± 40.7 ** |
| Isomer C | 0.3 mg/kg | 113 ± 19 | 31.4 ± 6.0 | 519.3 ± 33.7 |
| Isomer C | 1 mg/kg | 128 ± 24 | 30.3 ± 6.5 | 510.2 ± 44.9 |
| Isomer C | 3mg/kg | 125 ± 22 | 30.2 ± 5.5 | 493.6 ± 38.5 |
| Isomer | 10 mg/kg | 164 ± 30 | 42.7 ± 8.0 | 465.7 ± 49.0 |
| Isomer B | 0.3mg/kg | 101 ± 29 | 28.9 ± 9.2 | 566.4 ± 44.3 |
| Isomer B | 1 mglkg | 125 ± 18 | 34.5 ± 6.2 | 525.8 ± 28.6 |
| Isomer B | 3 mg/kg | 113 ± 17 | 31.1 ± 6.5 | 530.5 ± 38.0 |
| Isomer B | 10 mg/kg | 120 ± 26 | 30.9 ± 6.4 | 515.0 ± 53.0 |

| | | | | |
|---|---|---|---|---|
| **Significantly different from Vehicle group (p,0.01)ANOVA one way followed by Dunnett's test. | | | | |

The results demonstrate that tetrabenazine produces a dose-dependent sedative effect 45 minutes and 3 hours after administration whereas Isomer B and Isomer C show no sedative effects at any time, although isomer C does show a slight and non-significant hyperlocomotor effect 3 hours after administration..

### EXAMPLE 7

### Pharmaceutical Compositions

### (i) Tablet Formulation -I

A tablet composition containing a dihydrotetrabenazine of the invention is prepared by mixing 50mg of the dihydrotetrabenazine with 197mg of lactose (BP) as diluent, and 3mg magnesium stearate as a lubricant and compressing to form a tablet in known manner.

### (ii) Tablet Formulation - II

A tablet composition containing a dihydrotetrabenazine of the invention is prepared by mixing the compound (25 mg) with iron oxide, lactose, magnesium stearate, starch maize white and talc, and compressing to form a tablet in known manner.

### (iii) Capsule Formulation

A capsule formulation is prepared by mixing 100mg of a dihydroletrabenazine of the invention with 100mg lactose and filling the resulting mixture into standard opaque hard gelatin capsules.

## Claims

1. 3,11b-*cis*-dihydrotetrabenazine for use in halting or slowing the progress of one or more symptoms of Huntington's disease in a patient wherein the symptoms are selected from involuntary movements and degeneration in gait.

2. A compound for use according to claim 1 wherein the symptoms are involuntary movements selected from involuntary chorea, tremors and twitches.

3. 3,1 1b-*cis*-dihydrotetrabenazine for use in the prophylactic treatment of Huntington's disease in a patient identified as carrying the mutant gene responsible for Huntington's disease.

4. 3,11b-*cis*-dihydrotetrabenazine for use according to claim 3 wherein the 3,11b-cis-dihydrotetrabenazine is for use in the prophylactic treatment of a patient within the age range 15 - 50 years who is carrying the mutant form of the HD gene but who have not yet developed symptoms of the disease, the prophylactic treatment being for the purpose of preventing or slowing the onset of symptoms associated with Huntington's disease.

5. 3,11b-*cis*-dihydrotetrabenazine for use according to any one of claims 1 to 3 wherein the 3,11b-*cis*-dihydrotetrabenazine has an isomeric purity of greater than 90%.

6. 3,11b-*cis*-dihydrotetrabenazine for use according to claim 5 wherein the 3,11b-*cis*-dihydrotetrabenazine has an isomeric purity of greater than 95%.

7. 3,11b-*cis*-dihydrotetrabenazine for use according to claim 6 wherein the 3,11b-*cis*-dihydrotetrabenazine has an isomeric purity of greater than 98%.

8. 3,11b-*cis*-dihydrotetrabenazine for use according to any one of the preceding claims wherein the dihydrotetrabenazine is in the form of an acid addition salt.

9. 3,11b-*cis*-dihydrotetrabenazine for use according to claim 8 wherein the salt is a methane sulphonate salt.

10. 3,11b-*cis*-dihydrotetrabenazine for use according to any one of the preceding claims wherein a patient to whom the *cis*-dihydrotetrabenazine is administered carries a mutant form of the gene in which the number of CAG repeats on the IT-15 gene is at least thirty five.

11. 3,11b-*cis*-dihydrotetrabenazine for use according to any one of the preceding claims wherein a patient to whom the *cis*-dihydrotetrabenazine is administered carries a mutant form of the gene in which the number of CAG repeats on the IT-15 gene is at least forty.

12. 3,11b-*cis*-dihydrotetrabenazine for use according to any one of the preceding claims wherein a patient to whom the *cis*-dihydrotetrabenazine is administered carries a mutant form of the gene in which the number of CAG repeats on the IT-15 gene is at least 50.

13. A compound for use according to claim 3 wherein the prophylactic treatment is for preventing or slowing down sub-clinical progression of the disease in a patient.

14. The use of of 3,11b-*cis*-dihydrotetrabenazine or a pharmaceutically acceptable salt thereof for the manufacture of a medicament for a use as defmed in any one of claims 1 to 13.

## Patentansprüche

1. 3,11b-*cis*-Dihydrotetrabenazin zur Verwendung beim Stoppen oder Verlangsamen des Fortschreitens von einem oder mehreren Symptomen der Huntington-Krankheit bei einem Patienten, worin die Symptome aus unfreiwilligen Bewegungen und der Degeneration des Gangs ausgewählt werden.

2. Verbindung zur Verwendung nach Anspruch 1, worin die Symptome unfreiwillige Bewegungen sind, die aus unfreiwilliger Chorea, Tremores und Zucken ausgewählt sind.

3. 3,11b-*cis*-Dihydrotetrabenazin zur Verwendung bei der prophylaktischen Behandlung der Huntington-Krankheit bei einem Patienten, bei dem festgestellt wurde, dass er das für die Huntington-Krankheit verantwortliche mutante Gen trägt.

4. 3,11b-*cis*-Dihydrotetrabenazin zur Verwendung nach Anspruch 3, worin das 3,11b-*cis*-Dihydrotetrabenazin zur Verwendung bei der prophylaktischen Behandlung eines Patienten im Altersbereich von 15-50 Jahren ist, der die mutante Form des HD-Gens trägt, aber noch keine Symptome der Krankheit entwickelt hat, wobei die prophylaktische Behandlung zum Zweck der Vorbeugung oder Verlangsamung des Beginns der Symptome erfolgt, die mit der Huntington-Krankheit im Zusammenhang stehen.

5. 3,11b-*cis*-Dihydrotetrabenazin zur Verwendung nach einem der Ansprüche 1 bis 3, worin das 3,11b-*cis-*Dihydrotetrabenazin eine Isomerenreinheit von größer als 90 % aufweist.

6. 3,11b-*cis*-Dihydrotetrabenazin zur Verwendung nach Anspruch 5, worin das 3,11b-*cis*-Dihydrotetrabenazin eine Isomerenreinheit von größer als 95 % aufweist.

7. 3,11b-*cis*-Dihydrotetrabenazin zur Verwendung nach Anspruch 6, worin das 3,11b-*cis*-Dihydrotetrabenazin eine Isomerenreinheit von größer als 98 % aufweist.

8. 3,11b-*cis*-Dihydrotetrabenazin zur Verwendung nach einem der vorstehenden Ansprüche, worin das Dihydrotetrabenazin in Form eines Säureadditionssalzes vorliegt.

9. 3,11b-*cis*-Dihydrotetrabenazin zur Verwendung nach Anspruch 8, worin das Salz ein Methansulfonatsalz ist.

10. 3,11b-*cis*-Dihydrotetrabenazin zur Verwendung nach einem der vorstehenden Ansprüche, worin ein Patient, an den das *cis*-Dihydrotetrabenazin verabreicht wird, eine mutante Form des Gens trägt, in dem die Anzahl der CAG-Wiederholungen auf dem IT-15-Gen mindestens fünfunddreißig beträgt.

11. 3,11b-*cis*-Dihydrotetrabenazin zur Verwendung nach einem der vorstehenden Ansprüche, worin ein Patient, an den das *cis*-Dihydrotetrabenazin verabreicht wird, eine mutante Form des Gens trägt, in dem die Anzahl der CAG-Wiederholungen auf dem IT-15-Gen mindestens vierzig beträgt.

12. 3,11b-*cis*-Dihydrotetrabenazin zur Verwendung nach einem der vorstehenden Ansprüche, worin ein Patient, an den das *cis*-Dihydrotetrabenazin verabreicht wird, eine mutante Form des Gens trägt, in dem die Anzahl der CAG-Wiederholungen auf dem IT-15-Gen mindestens 50 beträgt.

13. Verbindung zur Verwendung nach Anspruch 3, worin die prophylaktische Behandlung zur Vorbeugung oder Verlangsamung des subklinischen Fortschreitens der Krankheit bei einem Patienten erfolgt.

14. Verwendung von 3,11b-*cis*-Dihydrotetrabenazin oder eines pharmazeutisch verträglichen Salzes davon für die Herstellung eines Medikaments für eine Verwendung wie sie in einem der Ansprüche 1 bis 13 definiert ist.

## Revendications

1. 3,11b-cis-dihydrotétrabénazine destinée à être utilisée dans l'arrêt ou le ralentissement de la progression d'un ou plusieurs symptômes de la maladie de Huntington chez un patient, dans laquelle les symptômes sont sélectionnés parmi des mouvements involontaires et la dégénération de la marche.

2. Composé destiné à être utilisée selon la revendication 1, dans lequel les symptômes sont des mouvements involontaires sélectionnés parmi une chorée involontaire, des tremblements et des secousses musculaires élémentaires.

3. 3,11b-cis-dihydrotétrabénazine destinée à être utilisée dans le traitement prophylactique de la maladie de Huntington chez un patient identifié comme portant le gène mutant responsable de la maladie de Huntington.

4. 3,11b-cis-dihydrotétrabénazine destinée à être utilisée selon la revendication 3, dans laquelle la 3,11b-cis-dihydrotétrabénazine est destinée à être utilisée dans le traitement prophylactique d'un patient au sein de la plage d'âge de 15 à 50 ans qui est porteur de la forme mutante du gène HD mais qui n'a pas encore développé de symptômes de la maladie, le traitement prophylactique étant dans le but d'empêcher ou de ralentir le déclenchement des symptômes associés à la maladie de Huntington.

5. 3,11b-cis-dihydrotétrabénazine destinée à être utilisée selon l'une quelconque des revendications 1 à 3, dans laquelle la 3,11b-cis-dihydrotétrabénazine possède une pureté isomère supérieure à 90 %.

6. 3,11b-cis-dihydrotétrabénazine destinée à être utilisée selon la revendication 5, dans laquelle la 3,11b-cis-dihydrotétrabénazine possède une pureté isomère supérieure à 95 %.

7. 3,11b-cis-dihydrotétrabénazine destinée à être utilisée selon la revendication 6, dans laquelle la 3,11b-cis-dihydrotétrabénazine possède une pureté isomère supérieure à 98 %.

8. 3,11b-cis-dihydrotétrabénazine destinée à être utilisée selon l'une quelconque des revendications précédentes, dans laquelle la dihydrotétrabénazine est sous la forme d'un sel d'addition acide.

9. 3,11b-cis-dihydrotétrabénazine destinée à être utilisée selon la revendication 8, dans laquelle le sel est un sel de méthanesulfonate.

10. 3,11b-cis-dihydrotetrabénazine destinée à être utilisée selon l'une quelconque des revendications précédentes, dans laquelle un patient auquel la cis-dihydrotétrabénazine est administrée porte une forme mutante du gène dans laquelle le nombre de répétitions CAG sur le gène IT-15 est d'au moins trente-cinq.

11. 3,11b-cis-dihydrotétrabénazine destinée à être utilisée selon l'une quelconque des revendications précédentes, dans laquelle un patient auquel la cis-dihydrotétrabénazine est administrée porte une forme mutante du gène dans laquelle le nombre de répétitions CAG sur le gène IT-15 est d'au moins quarante.

12. 3,11b-cis-dihydrotétrabénazine destinée à être utilisée selon l'une quelconque des revendications précédentes, dans laquelle un patient auquel la cis-dihydrotétrabénazine est administrée porte une forme mutante du gène dans laquelle le nombre de répétitions CAG sur le gène IT-15 est d'au moins 50.

13. Composé destiné à être utilisé selon la revendication 3, dans lequel le traitement prophylactique est pour empêcher ou ralentir la progression sous-clinique de la maladie chez un patient.

14. Utilisation de 3,11b-cis-dihydrotétrabénazine ou d'un sel pharmaceutiquement acceptable de celle-ci pour la fabrication d'un médicament destiné à être utilisé tel que défini dans l'une quelconque des revendications 1 à 13.
